# EUROPEAN PATENT SPECIFICATION

(11) **EP 3 015 449 B1**
(45) Date of publication and mention of the grant of the patent: **10.10.2018**
(21) Application number: 14816822.2
(22) Date of filing: 26.06.2014
(51) Int. Cl.: C07C 67/03, C07C 67/54, C07C 69/54, B01D 3/00

(54) **(METH)ACRYLATE PRODUCTION SYSTEM**
(METH)ACRYLAT-PRODUKTIONSSYSTEM
SYSTÈME DE PRODUCTION D'UN (MÉTH)ACRYLATE

(30) Priority: 27.06.2013 JP 2013134582
(43) Date of publication of application: 04.05.2016
(73) Proprietor: Osaka Organic Chemical Ind., Ltd., Osaka-shi Osaka 541-0052 (JP)
(72) Inventor: MATSUMOTO, Shigeaki, Kashiwara-shi Osaka 582-0020 (JP); ITO, Keisuke, Kashiwara-shi Osaka 582-0020 (JP); MATSUNO, Masayoshi, Kashiwara-shi Osaka 582-0020 (JP)
(74) Representative: Neymeyr, Ulrich Theodor Paul
(86) International application number: PCT/JP2014/067043
(87) International publication number: WO 2014/208686

(56) References cited:
- DE-A1- 10 063 510
- JP-A- H08 268 938
- JP-A- H08 268 938
- JP-B2- 4 520 310

## Description

### TECHNICAL FIELD

The present invention relates to a system for producing a (meth)acrylate. More specifically, the present invention relates to a system for producing a (meth)acrylate, which enables to efficiently produce a (meth)acrylate with transesterification. The (meth)acrylate is a useful compound, for example, as a raw material of a (meth)acrylic resin, a surfactant, an adhesive, a paint and the like, the kind of which differs depending on the kind of the (meth)acrylate.

In this description, the term "(meth)acrylate" means "acrylate" and/or "methacrylate".

### BACKGOUND ART

As a method for efficiently separating methanol from a mixture of methyl (meth)acrylate and methanol by using a reactor having a distillation column, there has been proposed a method which includes using an azeotropic solvent which forms an azeotropic mixture with methanol, refluxing a part of a condensate of a vapor, which is distilled out from the top of the distillation column, to the distillation column, separating the remaining condensate into two layers, supplying the upper layer of the two layers to a middle stage of the distillation column, taking out the lower layer of the remaining condensate from the distillation column, and collecting methyl (meth)acrylate from the bottom of the reactor (for example, see Patent Literature 1).

According to the above-mentioned method, methyl (meth)acrylate can be collected from the bottom of the reactor. However, after the collection of the methyl (meth)acrylate, methyl (meth)acrylate remaining in the reactor cannot be collected.

As a method for producing a (meth)acrylate as an objective compound by using a transesterification reaction of an alkyl (meth)acrylate used as a raw material and an alcohol, there has been proposed a method for producing a (meth)acrylate which includes, in carrying out a transesterification reaction in the presence of an azeotropic solvent which forms an azeotropic mixture with an alkyl alcohol which is generated as a by-product, removing the alkyl alcohol generated as a by-product together with the azeotropic solvent from a distillation outlet provided at the upper part of the distillation column, controlling the temperature of vapor distilled out from the distillation outlet to a temperature not lower than the azeotropic temperature of the alkyl alcohol which is generated as a by-product and the azeotropic solvent, and a temperature not higher than a temperature 2°C higher than the azeotropic temperature, and controlling the temperature at the bottom of the distillation column to a temperature not lower than a temperature 10°C lower than the boiling point of the azeotropic solvent, and a temperature not higher than the boiling point of the azeotropic solvent (for example, see Patent Literature 2).

According to the above-mentioned method for producing a (meth)acrylate, it has been considered that a Michael addition reaction which is a side reaction can be inhibited, and that a (meth)acrylate can be produced in high productivity. However, an alkyl (meth)acrylate used as a raw material remains in the reaction system after an objective (meth)acrylate is produced, and there has not yet been considered a system which enables to efficiently collect the remaining alkyl (meth)acrylate used as a raw material in the reaction system.

Therefore, it has been desired to develop a system for producing a (meth)acrylate with transesterification, which enables to efficiently collect and reuse a (meth)acrylate which is used as a raw material, a solvent, an alcohol generated as a by-product and the like, remaining in a reaction system after an objective (meth)acrylate is produced by transesterification, and which enables to efficiently produce an objective (meth)acrylate.

### PRIOR ART LITERATURES

### PATENT LITERATURES

Patent Literature 1: Japanese Unexamined Patent Publication No. Hei 8-268938
Patent Literature 2: Japanese Unexamined Patent Publication No. 2004-189650

### SUMMARY OF THE INVENTION

### PROBLEMS TO BE SOLVED BY THE INVENTION

The present invention has been achieved in view of the above-mentioned prior arts. An object of the present invention is to provide a system for producing a (meth)acrylate by means of transesterification, which enables to efficiently collect and reuse a (meth)acrylate which is used as a raw material, a solvent, an alcohol generated as a by-product and the like, remaining in a reaction system after an objective (meth)acrylate is produced by transesterification, and which enables to efficiently produce an objective (meth)acrylate.

### MEANS FOR SOLVING THE PROBLEMS

The present invention relates to
(1) a system (an apparatus) for producing a (meth)acrylate used in producing a (meth)acrylate by transesterification, which includes a reactor A having a distillation column and a distillation apparatus B having a distillation column, wherein
   an upper part of the distillation column of the reactor A is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a liquid separation apparatus through the switching apparatus with a pipe for feeding the condensate remaining in the condensing apparatus to the liquid separation apparatus;
   an upper part of the liquid separation apparatus is connected with the distillation column through a pipe for refluxing an upper layer of the condensate separated by the liquid separation apparatus to the distillation column; and a lower part of the liquid separation apparatus is connected with the distillation apparatus B through a pipe for feeding a lower layer of the condensate separated by the liquid separation apparatus to the distillation apparatus B;
   an upper part of the distillation column of the distillation apparatus B is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a switching apparatus with a pipe for feeding the remaining condensate to the collecting unit; and
   a lower part of the distillation apparatus B is connected with a pipe between the switching apparatus and the liquid separation apparatus in the reactor A through a pipe for refluxing a residue existing in the distillation apparatus B to the distillation column of the reactor A; and
(2) a system (an apparatus) for producing a (meth)acrylate used in producing a (meth)acrylate by transesterification, which includes a reactor A having a distillation column, a distillation apparatus B having a distillation column, and a distillation apparatus C having a distillation column, wherein
   an upper part of the distillation column of the reactor A is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a liquid separation apparatus through a switching apparatus with a pipe for feeding the condensate remaining in the condensing apparatus to the liquid separation apparatus;
   an upper part of the liquid separation apparatus is connected with the distillation column through a pipe for refluxing an upper layer of the condensate separated by the liquid separation apparatus to the distillation column; and a lower part of the liquid separation apparatus is connected with the distillation apparatus B through a pipe for feeding a lower layer of the condensate separated by the liquid separation apparatus to the distillation apparatus B;
   an upper part of the distillation column of the distillation apparatus B is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a switching apparatus with a pipe for feeding the remaining condensate to the collecting unit;
   a lower part of the distillation apparatus B is connected with the distillation apparatus C through a pipe for feeding a residue existing in the distillation apparatus B to the distillation column of the distillation apparatus C;
   an upper part of the distillation apparatus C is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a pipe for feeding the condensate remaining in the condensing apparatus to the collecting unit; and
   a lower part of the distillation apparatus C is connected with a pipe between the switching apparatus and the liquid separation apparatus in the reactor A through a pipe for feeding a residue existing in the distillation apparatus C to the distillation column of the reactor A.

### EFFECTS OF THE INVENTION

According to the system for producing a (meth)acrylate of the present invention, there are exhibited excellent effects such that a (meth)acrylate which is used as a raw material, a solvent, an alcohol which is generated as a by-product and the like, remaining in a reaction system after producing an objective (meth)acrylate by transesterification can be efficiently collected and reused, and an objective (meth)acrylate can be efficiently produced.

### BRIEF DESCRIPTION OF DRAWINGS

[Fig. 1] Fig. 1 is a schematic drawing showing one embodiment of a system for producing a (meth)acrylate according to the present invention.
[Fig. 2] Fig. 2 is a schematic drawing showing another embodiment of a system for producing a (meth)acrylate according to the present invention.

### MODES FOR CARRYING OUT THE INVENTION

Generally, a (meth)acrylate such as methyl (meth)acrylate is sensitive to a temperature, and easily polymerizes. Therefore, in order to inhibit the polymerization of the (meth)acrylate, it can be considered to use a system for collecting a (meth)acrylate which is used as a raw material remaining in a reactor by reduced-pressure distillation at a low temperature.

However, when the (meth)acrylate is collected by reduced-pressure distillation at a low temperature, in the case where the boiling point of the (meth)acrylate is low, and the vapor pressure of the (meth)acrylate is high, it is difficult to trap the vapor of the (meth)acrylate with a condensing apparatus, and the vapor is scattered to the air. Therefore, the (meth)acrylate cannot be efficiently collected, and there is a possibility that an offensive odor of the (meth)acrylate is diffused to the air.
In order to avoid the diffusion of the vapor of the (meth)acrylate to the air, it can be considered to use an apparatus for treating exhaust gas in the condensing apparatus. However, when the apparatus for treating exhaust gas is used, there arise some secondary problems such as treatment of waste liquid which is generated from the apparatus for treating exhaust gas, and increase in costs such as equipment costs for use of an apparatus for treating exhaust gas and maintenance costs of the apparatus for treating exhaust gas.

Therefore, the present inventors have earnestly studied to overcome the above problems. As a result, when a system for producing a (meth)acrylate which is functionally constructed by using a reactor A having a distillation column and a distillation apparatus B having a distillation column, or a system for producing a (meth)acrylate which is functionally constructed by using a reactor A having a distillation column, a distillation apparatus B having a distillation column and a distillation apparatus C having a distillation column, a (meth)acrylate which is used as a raw material remaining in the reactor A can be efficiently collected from the reactor A under atmospheric pressure without an operation for reduced pressure as mentioned above; a solvent, an alcohol generated as a by-product and the like can be efficiently collected and reused; and an objective (meth)acrylate can be efficiently produced. Furthermore, it has also been found out that the collected (meth)acrylate used as a raw material can be suitably used as a raw material when a new transesterification reaction of a material (meth)acrylate and an alcohol corresponding to an objective (meth)acrylate is carried out.

Thus, according to the system for producing a (meth)acrylate of the present invention, there is no necessity to use an operation for reducing a pressure; a (meth)acrylate used as a raw material remaining in a reactor A can be efficiently collected under atmospheric pressure; a solvent, an alcohol generated as a by-product and the like can be efficiently collected and reused; an objective (meth)acrylate can be efficiently produced; and moreover the collected (meth)acrylate can be reused.

Therefore, the system for producing a (meth)acrylate of the present invention is a system for producing a (meth)acrylate in high industrial productivity.

As described above, the system for producing a (meth)acrylate of the present invention is a system for producing a (meth)acrylate when a (meth)acrylate is produced by transesterification.

The system for producing a (meth)acrylate of the present invention has a reactor A having a distillation column and a distillation apparatus B having a distillation column. An upper part of the distillation column of the reactor A is connected with a condensing apparatus through a pipe, the condensing apparatus is connected with the upper part of the distillation column through a switching apparatus with a pipe for refluxing a part of a condensate obtained in the condensing apparatus to the upper part of the distillation column, and the condensing apparatus is connected with a liquid separation apparatus through the switching apparatus with a pipe for feeding the condensate remaining in the condensing apparatus to the liquid separation apparatus; an upper part of the liquid separation apparatus is connected with the distillation column through a pipe for refluxing an upper layer of the condensate separated by the liquid separation apparatus to the distillation column, and a lower part of the liquid separation apparatus is connected with the distillation apparatus B through a pipe for feeding a lower layer of the condensate separated by the liquid separation apparatus to the distillation apparatus B; an upper part of the distillation column of the distillation apparatus B is connected with a condensing apparatus through a pipe, the condensing apparatus is connected with the upper part of the distillation column through a switching apparatus with a pipe for refluxing a part of a condensate obtained in the condensing apparatus to the upper part of the distillation column, and the condensing apparatus is connected with a collecting unit for collecting the condensate remaining in the condensing apparatus through a switching apparatus with a pipe for feeding the remaining condensate to the collecting unit; and a lower part of the distillation apparatus B is connected with a pipe between the switching apparatus and the liquid separation apparatus in the reactor A through a pipe for refluxing a residue existing in the distillation apparatus B to the distillation column of the reactor

In addition, the system for producing a (meth)acrylate of another embodiment of the present invention has a reactor A having a distillation column, a distillation apparatus B having a distillation column and a distillation apparatus C having a distillation column. An upper part of the distillation column of the reactor A is connected with a condensing apparatus through a pipe, the condensing apparatus is connected with the upper part of the distillation column through a switching apparatus with a pipe for refluxing a part of a condensate obtained in the condensing apparatus to the upper part of the distillation column, and the condensing apparatus is connected with a liquid separation apparatus through a switching apparatus with a pipe for feeding the condensate remaining in the condensing apparatus to the liquid separation apparatus; an upper part of the liquid separation apparatus is connected with the distillation column through a pipe for refluxing an upper layer of the condensate separated by the liquid separation apparatus to the distillation column, and a lower part of the liquid separation apparatus is connected with the distillation apparatus B through a pipe for feeding a lower layer of the condensate separated by the liquid separation apparatus to the distillation apparatus B; an upper part of the distillation column of the distillation apparatus B is connected with a condensing apparatus through a pipe, the condensing apparatus is connected with the upper part of the distillation column through a switching apparatus with a pipe for refluxing a part of a condensate obtained in the condensing apparatus to the upper part of the distillation column, and the condensing apparatus is connected with a collecting unit for collecting the condensate remaining in the condensing apparatus through a switching apparatus with a pipe for feeding the remaining condensate to the collecting unit; a lower part of the distillation apparatus B is connected with the distillation apparatus C through a pipe for feeding a residue existing in the distillation apparatus B to the distillation column of the distillation apparatus C; an upper part of the distillation apparatus C is connected with a condensing apparatus through a pipe; the condensing apparatus is connected with the upper part of the distillation column through a switching apparatus with a pipe for refluxing a part of a condensate obtained in the condensing apparatus to the upper part of the distillation column, and the condensing apparatus is connected with a collecting unit for collecting the condensate remaining in the condensing apparatus through a pipe for feeding the condensate remaining in the condensing apparatus to the collecting unit; and a lower part of the distillation apparatus C is connected with a pipe between the switching apparatus and the liquid separation apparatus in the reactor A through a pipe for feeding a residue existing in the distillation apparatus C to the distillation column of the reactor A.

In the system for producing a (meth)acrylate of the present invention, a (meth)acrylate which is used as a raw material includes, for example, an alkyl (meth)acrylate having an alkyl group of 1 to 8 carbon atoms, such as methyl (meth)acrylate, and the like, and the present invention is not limited only to those exemplified ones.

A transesterification of the (meth)acrylate which used as a raw material and an alcohol corresponding to an objective (meth)acrylate can be carried out by any one of a flow method and a batch method.

In the present invention, first of all, a transesterification of the (meth)acrylate which used as a raw material and an alcohol corresponding to an objective (meth)acrylate is carried out.

The alcohol which is used as a raw material in carrying out a transesterification of the (meth)acrylate is appropriately selected in accordance with an objective (meth)acrylate. More specifically, as the above-mentioned alcohol, an alcohol which forms an ester group of an objective (meth)acrylate is used. As one example, when an objective (meth)acrylate is, for example, n-propyl (meth)acrylate, n-propyl alcohol is used as an alcohol which forms the propyl group.

The alcohol corresponding to an objective (meth)acrylate includes, for example, an aliphatic or alicyclic alcohol represented by the formula (I):
[Chem. 1]

R¹OH (I)

wherein R¹ is an alkyl group having 2 to 30 carbon atoms which may have a cyclic structure, such as ethyl alcohol, n-propyl alcohol, isopropyl alcohol, n-butyl alcohol, isobutyl alcohol, sec-butyl alcohol, tert-butyl alcohol, n-pentyl alcohol, n-hexyl alcohol, isohexyl alcohol, cyclohexyl alcohol, 3,3,5-trimethylcyclohexyl alcohol, 4-tert-butylcyclohexyl alcohol, n-heptyl alcohol, n-octyl alcohol, isooctyl alcohol, 2-ethylhexyl alcohol, 3,4-dimethylhexyl alcohol, 3,4-dimethylheptyl alcohol, lauryl alcohol, nonyl alcohol, isononyl alcohol, stearyl alcohol or 2-heptylundecan-1-ol; an aromatic alcohol such as phenol, benzyl alcohol, 1-phenylethyl alcohol, 2-phenylethyl alcohol or phenoxyethanol; an amino alcohol represented by the formula (II): 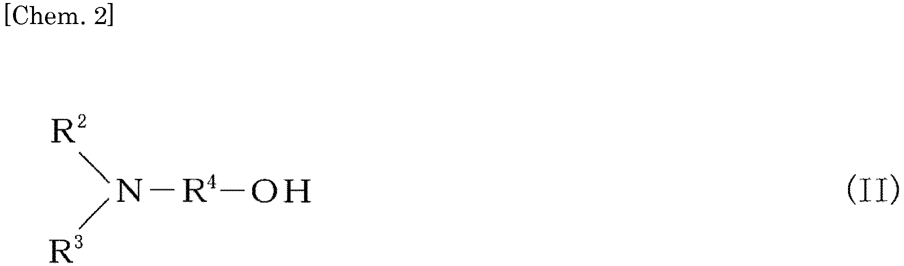 wherein each of R² and R³ is independently hydrogen atom or an alkyl group having 1 to 8 carbon atoms, and R⁴ is an alkylene group having 1 to 4 carbon atoms, such as dimethylaminoethyl alcohol, diethylaminoethyl alcohol, dipropylaminoethyl alcohol, dibutylaminoethyl alcohol, dipentylaminoethyl alcohol, dihexylaminoethyl alcohol, dioctylaminoethyl alcohol, methylethylaminoethyl alcohol, methylpropylaminoethyl alcohol, methylbutylaminoethyl alcohol, methylhexylaminoethyl alcohol, ethylpropylaminoethyl alcohol, ethylbutylaminoethyl alcohol, ethylpentylaminoethyl alcohol, ethyloctylaminoethyl alcohol, propylbutylaminoethyl alcohol, dimethylaminopropyl alcohol, diethylaminopropyl alcohol, dipropylaminopropyl alcohol, dibutylaminopropyl alcohol or butylpentylaminopropyl alcohol; an alkoxy alcohol such as 2-methoxyethyl alcohol, ethoxyethyl alcohol, butoxyethyl alcohol, ethylene glycol monomethyl ether, ethylene glycol monoethyl ether (ethyl carbitol), triethylene glycol monomethyl ether, tetrahydrofurfuryl alcohol, 2-ethyl-2-methyl-1,3-dioxolane-4-methyl alcohol, cyclohexanespiro-2-1,3-dioxolane-4-methyl alcohol, 3-ethyl-3-oxetanylmethyl alcohol, or 3-ethyl-3-oxetanylmethyl alcohol; a monohydric alcohol such as allyl alcohol, methallyl alcohol or tetrahydrofurfuryl alcohol; a dihydric alcohol such as ethylene glycol, 2,3-propanediol, 1,3-butanediol, 1,4-butanediol, 1,6-hexanediol, trimethylolpropane or cyclohexanediol; and a polyhydric alcohol having at least three hydroxyl groups such as glycerol, and the like, and the present invention is not limited only to those exemplified ones. These alcohols can be used alone, or at least two kinds thereof can be used in combination.

The amount of the (meth)acrylate which is used as a raw material is preferably 0.3 equivalent or more, more preferably 0.5 equivalent or more and furthermore preferably 0.8 equivalent or more per one equivalent of hydroxyl group of an alcohol corresponding to an objective (meth)acrylate from the viewpoint of increase in reaction rate of transesterification of the (meth)acrylate which is used as a raw material and the alcohol, and is preferably 5 equivalents or less, more preferably 4 equivalents or less and furthermore preferably 3 equivalents or less per one equivalent of hydroxyl group of the alcohol corresponding to an objective (meth)acrylate from the viewpoint of reduction of the amount of unreacted (meth)acrylate which is used as a raw material.

A preferred (meth)acrylate which is used as a raw material includes methyl (meth)acrylate.

When the transesterification of the (meth)acrylate which is used as a raw material and an alcohol corresponding to an objective (meth)acrylate is carried out, a transesterification catalyst can be used.

The transesterification catalyst includes, for example, hydroxides such as lithium hydroxide, sodium hydroxide, potassium hydroxide, cesium hydroxide, magnesium hydroxide, calcium hydroxide, barium hydroxide and strontium hydroxide; hydrogen carbonates such as lithium hydrogen carbonate, sodium hydrogen carbonate, potassium hydrogen carbonate, cesium hydrogen carbonate, magnesium hydrogen carbonate, calcium hydrogen carbonate, barium hydrogen carbonate and strontium hydrogen carbonate; carbonates such as lithium carbonate, sodium carbonate, potassium carbonate, cesium carbonate, magnesium carbonate, calcium carbonate, barium carbonate and strontium carbonate; acetates such as lithium acetate, sodium acetate, potassium acetate, cesium acetate, magnesium acetate, calcium acetate, barium acetate and strontium acetate; borohydrides such as lithium borohydride, sodium borohydride, potassium borohydride and cesium borohydride; salts of stearic acid such as lithium stearate, sodium stearate, potassium stearate, cesium stearate, magnesium stearate, calcium stearate, barium stearate and strontium stearate; phenylborates such as lithium phenylborate, sodium phenylborate, potassium phenylborate and cesium phenylborate; benzoates such as lithium benzoate, sodium benzoate, potassium benzoate and cesium benzoate; hydrogenphosphates such as dilithium hydrogenphosphate, disodium hydrogenphosphate, dipotassium hydrogenphosphate and dicesium hydrogenphosphate; phenylphosphates such as dilithium phenylphosphate, disodium phenylphosphate, dipotassium phenylphosphate and dicesium phenylphosphate; metal alkoxides such as sodium alkoxide and titanium alkoxide; tetraalkoxytitaniums such as tetramethoxytitanium, tetraethoxytitanium, tetrapropoxytitanium and tetrabutoxytitanium; dialkyltin oxides such as dialkyltin oxides having an alkyl group of 4 to 18 carbon atoms, such as dibutyltin oxide, dioctyltin oxide and dilauryltin oxide; metal alcoholates such as titanium alcoholate, aluminum alcoholate and magnesium alcoholate, and the like, and the present invention is not limited only to those exemplified ones. These transesterification catalysts can be used alone, or at least two kinds thereof can be used in combination. Among these transesterification catalysts, a tetraalkoxytitanium and a dialkyltin oxide having an alkyl group of 4 to 12 carbon atoms are preferable, a tetraalkoxytitanium and a dialkyltin oxide having an alkyl group of 4 to 8 carbon atoms are more preferable, and tetramethoxytitanium, dibutyltin oxide and dioctyltin oxide are furthermore preferable, from the viewpoint of acceleration of transesterification.

The amount of the transesterification catalyst cannot be absolutely determined since the amount differs depending on the kind of the transesterification catalyst. Therefore, it is preferred that the amount of the transesterification catalyst is appropriately determined in accordance with the kind of the transesterification catalyst. The amount of the transesterification catalyst is usually preferably 0.00001 moles or more, more preferably 0.0001 moles or more per one mole of the (meth)acrylate which is used as a raw material, from the viewpoint of efficient progress of transesterification of the (meth)acrylate which is used as a raw material and the alcohol, and is preferably 0.10 moles or less, more preferably 0.05 moles or less per one mole of the (meth)acrylate which is used as a raw material, from the viewpoint of improvement in economic efficiency.

When the transesterification reaction of the (meth)acrylate which is used as a raw material and an alcohol corresponding to an objective (meth)acrylate is carried out, a polymerization inhibitor also can be used. The polymerization inhibitor includes, for example, N-oxyradical compounds such as 4-hydroxy-2,2,6,6-tetramethylpiperidine-N-oxyl, 4-acetoamino-2,2,6,6-tetramethylpiperidine-N-oxyl, 4-benzoyloxy-2,2,6,6-tetramethylpiperidine-N-oxyl, 4-oxo-2,2,6,6-tetramethylpiperidine-N-oxyl and 2,2,6,6-tetramethylpiperidine-N-oxyl; phenol compounds such as paramethoxyphenol, 2,2'-methylenebis(4-ethyl-6-tert-butylphenol), 2,6-di-tert-butyl-4-methylphenol, 2,6-di-tert-butyl-N,N-dimethylamino-p-cresol, 2,4-dimethyl-6-tert-butylphenol, 4-tert-butylcatechol, 4,4'-thio-bis(3-methyl-6-tert-butylphenol) and 4,4'-butylidene-bis(3-methyl-6-tert-butylphenol); quinone compounds such as methoquinone, hydroquinone, 2,5-di-tert-butylhydroquinone, 2,6-di-tert-butylhydroquinone and benzoquinone; copper dialkyldithiocarbamate such as cuprous chloride and copper dimethyldithiocarbamate; amino compounds such as phenothiazine, N,N'-diphenyl-p-phenylenediamine, phenyl-p-naphthylamine, N,N'-di-β-naphthyl-p-phenylenediamine and N-phenyl-N'-isopropyl-p-phenylenediamine; hydroxyamine compounds such as 1,4-dihydroxy-2,2,6,6-tetramethylpiperidine, 1-hydroxy-2,2,6,6-tetramethylpiperidine and 4-hydroxy-2,2,6,6-tetramethylpiperidine, and the like, and the present invention is not limited only to those exemplified ones. These polymerization inhibitors can be used alone, or at least two kinds thereof can be used in combination.

The amount of the polymerization inhibitor is preferably 0.00001 parts by mass or more, more preferably 0.00005 parts by mass or more, furthermore preferably 0.0001 parts by mass or more per 100 parts by mass of the (meth)acrylate which is used as a raw material, from the viewpoint of inhibition of polymerization of the (meth)acrylate which is used as a raw material and an objective (meth)acrylate, and is preferably 0.1 parts by mass or less, more preferably 0.05 parts by mass or less, furthermore preferably 0.01 parts by mass or less per 100 parts by mass of the (meth)acrylate which is used as a raw material, from the viewpoint of increase in purity of an objective (meth)acrylate.

When the transesterification reaction of the (meth)acrylate which is used as a raw material and the alcohol corresponding to an objective (meth)acrylate is carried out in the reactor A, it is preferred as a solvent to use an azeotropic solvent which forms an azeotropic mixture together with an alcohol which is generated as a by-product (hereafter referred to as by-product alcohol) by transesterification of the (meth)acrylate which is used as a raw material and the alcohol corresponding to an objective (meth)acrylate at a temperature equal to or lower than the boiling point of the by-product alcohol, and which forms an azeotropic mixture together with the (meth)acrylate which is used as a raw material at a temperature equal to or lower than the boiling point of the (meth)acrylate which is used as a raw material.

When the azeotropic solvent which forms an azeotropic mixture together with the by-product alcohol at a temperature equal to or lower than the boiling point of the by-product alcohol, and which forms an azeotropic mixture together with the (meth)acrylate which is used as a raw material at a temperature equal to or lower than the boiling point of the (meth)acrylate which is used as a raw material is used as a solvent, after the transesterification reaction of the (meth)acrylate which is used as a raw material and the alcohol corresponding to an objective (meth)acrylate is carried out, the (meth)acrylate which is used as a raw material existing in the reactor A can be efficiently collected from the reactor A.

When the (meth)acrylate which is used as a raw material is, for example, methyl (meth)acrylate, the by-product alcohol is methanol. In this case, as the azeotropic solvent which forms an azeotropic mixture together with the by-product alcohol at a temperature equal to or lower than the boiling point of the by-product alcohol, and which forms an azeotropic mixture together with the (meth)acrylate which is used as a raw material at a temperature equal to or lower than the boiling point of the (meth)acrylate which is used as a raw material, there can be exemplified cyclohexane, n-hexane and the like, and the present invention is not limited only to those exemplified ones. These azeotropic solvents can be used alone, or at least two kinds thereof can be used in combination. Among these azeotropic solvents, cyclohexane is preferable from the viewpoint of shortening of a period of time necessary for transesterification, and efficient collection of the (meth)acrylate which is used as a raw material.

The amount of the solvent is not particularly limited, and can be usually 5 to 200 parts by mass or so per 100 parts by mass of the sum of the (meth)acrylate which is used as a raw material and the alcohol corresponding to an objective (meth)acrylate.

Incidentally, within a scope which would not hinder an object of the present invention, other solvent can be used. The other solvent includes, for example, aliphatic hydrocarbon compounds having 5 to 8 carbon atoms other than cyclohexane, such as n-pentane, n-hexane, isohexane, n-heptane, n-octane, 2,3-dimethybutane, 2,5-dimethylhexane and 2,2,4-trimethylpentane, and the like, and the present invention is not limited only to those exemplified ones.

Next, the system for producing a (meth)acrylate of the present invention will be more specially described based on drawings. However, the present invention is not limited only to the embodiments shown in the drawings.

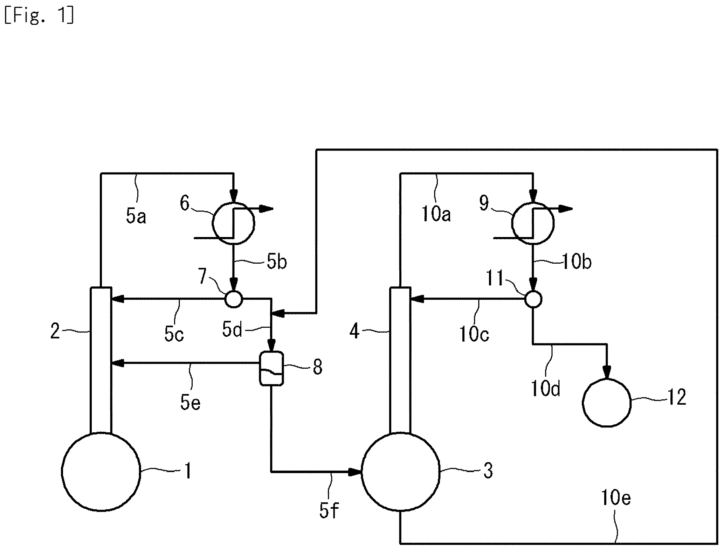

Fig. 1 is a schematic drawing which shows one embodiment of a system for producing a (meth)acrylate according to the present invention.

The system for producing a (meth)acrylate shown in Fig. 1 has a reactor A1 having a distillation column 2 and a distillation apparatus B3 having a distillation column 4.

In the present invention, the reactor A1 having the distillation column 2 is used when the transesterification of the (meth)acrylate used as a raw material and the alcohol corresponding to an objective (meth)acrylate is carried out.

The reactor A1 having the distillation column 2 includes, for example, a reactor having a distillation column such as a rectification column, a fluidized bed, a fixed bed or a reaction distillation column, and the like, and the present invention is not limited only to those exemplified ones. In addition, the structure and type of the distillation column 2 are not particularly limited. Among distillation columns 2, a distillation column having a high efficiency for gas-liquid contact is preferable. A suitable distillation column 2 includes, for example, a packed column type distillation column, a tray type distillation column and the like. The tray type distillation column includes, for example, a packed distillation column, an Oldershaw type distillation column, a lift-tray type distillation column and the like, and the present invention is not limited only to those exemplified ones. The number of theoretical stages of the distillation column 2 is preferably 7 or more, more preferably 10 or more, furthermore preferably 15 or more, from the viewpoint of efficient and stable progress of the transesterification of the (meth)acrylate used as a raw material and the alcohol corresponding to an objective (meth)acrylate, and is preferably 100 or less, more preferably 70 or less, furthermore preferably 50 or less, from the viewpoint of improvement in economic efficiency.

A condensing apparatus 6 is connected with the distillation column 2 of the reactor A1 at an upper part of the distillation column 2, preferably at the top of the distillation column 2 through a pipe 5a. In addition, in order to reflux a part of a condensate obtained in the condensing apparatus 6 to an upper part of the distillation column 2, preferably the top of the distillation column 2, the condensing apparatus 6 is connected with a switching apparatus 7 through a pipe 5b, and the switching apparatus 7 is connected with the distillation column 2 at an upper part of the distillation column 2, preferably the top of the distillation column 2 through a pipe 5c. The condensing apparatus 6 includes, for example, a condenser and the like, and the present invention is not limited only to the exemplified one. The switching apparatus 7 is used in order to feed the condensate obtained in the condensing apparatus 6 to the upper part, preferably the top of the distillation column 2 or a liquid separation apparatus 8. The switching apparatus 7 includes, for example, a switching valve, a reflux distributor, a solenoid valve and the like, and the present invention is not limited only to those exemplified ones.

In order to feed the condensate remaining in the condensing apparatus 6 to the liquid separation apparatus 8, the condensing apparatus 6 is connected with the switching apparatus 7 through the pipe 5b, and the switching apparatus 7 is connected with the liquid separation apparatus 8 through a pipe 5d. Therefore, a part of the condensate obtained in the condensing apparatus 6 is fed to the upper part, preferably the top of the distillation column 2 through the switching apparatus 7, and the remaining condensate is fed to the liquid separation apparatus 8 by switching of the switching apparatus 7. The liquid separation apparatus 8 is used when the condensate is separated into two layers of an upper layer and a lower layer. The liquid separation apparatus 8 includes, for example, a decanter, a separating funnel and an oil-water separating apparatus and the like, and the present invention is not limited only to those exemplified ones.

The upper part of the liquid separation apparatus 8 is connected with the distillation column 2 through a pipe 5e in order to reflux the upper layer of the condensate separated by the liquid separation apparatus 8 to the distillation column 2. The position where the pipe 5e is connected with the distillation column 2 is not particularly limited, and it is preferred that the pipe 5e is connected with the distillation column 2 at a middle stage from the viewpoint of an efficient transesterification reaction of the (meth)acrylate which is used as a raw material and an alcohol corresponding to an objective (meth)acrylate. The middle stage of the distillation column 2 means a central position of a multistage distillation column. When the distillation column 2 is, for example, a distillation column having a number of theoretical stages of 15, 6 to 9 stages of the distillation column correspond to the middle stage of the distillation column 2.

The lower part of the liquid separation apparatus 8 is connected with the distillation apparatus B3 through a pipe 5f in order to feed the lower layer of the condensate separated by the liquid separation apparatus 8 to the distillation apparatus B3.

The distillation apparatus B3 having the distillation column 4 can be any one of a batch type distillation apparatus and a flow type distillation apparatus. The distillation column 4 of the distillation apparatus B3 includes, for example, a packed column, a tray type distillation column and the like, and the present invention is not limited only to those exemplified ones. A filler used in the packed column includes, for example, Helipack, McMahon, Cascade Mini Ring and the like, and the present invention is not limited only to those exemplified ones. The tray type distillation column includes, for example, a packed distillation column, an Oldershaw distilling column, a Lift Tray distillation column and the like, and the present invention is not limited only to those exemplified ones.

The upper part, preferably the top of the distillation column 4 of the distillation apparatus B3 is connected with a condensing apparatus 9 through a pipe 10a. In order to reflux a part of the condensate obtained in the condensing apparatus 9 to the upper part, preferably the top of the distillation column 4, the condensing apparatus 9 is connected with a switching apparatus 11 through a pipe 10b, and the switching apparatus 11 is connected with the upper part, preferably the top of the distillation column 4 through a pipe 10c.

In order to collect the condensate remaining in the condensing apparatus 9, the condensing apparatus 9 is connected with the switching apparatus 11 through the pipe 10b, and the switching apparatus 11 is connected with a collecting unit 12 through a pipe 10d. Therefore, a part of the condensate obtained in the condensing apparatus 9 is fed to the upper part, preferably the top of the distillation column 4, and the remaining condensate is fed to the collecting unit 12 by switching the switching apparatus 11. The switching apparatus 11 includes, for example, a switching valve, a reflux distributor, a solenoid valve and the like, and the present invention is not limited only to those exemplified ones. In addition, the collecting unit 12 can be a so-called receiver.

A pipe 10e is connected with the lower part of the distillation apparatus B3 in order to reflux a residue existing in the distillation apparatus B3 to the distillation column 2 of the reactor A1. The other end of the pipe 10e is connected with the pipe 5d positioning between the switching apparatus 7 of the reactor A1 and the liquid separation apparatus 8 through, for example, a three-way cock (not illustrated in the figure) or the like.

It is preferred that vapor of a by-product alcohol is removed from the upper part, preferably the top of the distillation column 2 of the reactor A1 by carrying out the azeotrope of a solvent and the by-product alcohol from the viewpoint of an efficient transesterification reaction of the (meth)acrylate which is used as a raw material and an alcohol corresponding to an objective (meth)acrylate. It is preferred that the temperature at the top of the distillation column 2 is controlled so as to be within a temperature range from the azeotropic temperature of the by-product alcohol and the solvent to a temperature which is 5°C higher than the azeotropic temperature, preferably a temperature which is 2°C higher than the azeotropic temperature, from the viewpoint of efficient removal of the solvent and the by-product alcohol from the reactor A1.

The by-product alcohol can be collected as a condensate by taking out vapor from the upper part of the distillation column 2, and condensing the vapor by means of the condensing apparatus 6 while the transesterification reaction of the (meth)acrylate which is used as a raw material and an alcohol corresponding to an objective (meth)acrylate is carried out. A part of the condensate obtained in the condensing apparatus 6 is refluxed to the distillation column 2 by operating the switching apparatus 7 so as to connect the pipe 5b with the pipe 5c. The remaining condensate is removed from the reactor A1 through the liquid separation apparatus 8 by operating the switching apparatus 7 so as to connect the pipe 5b with the pipe 5d. The temperature of the upper part of the distillation column 2 can be easily controlled to an azeotropic temperature of the by-product alcohol and the solvent by operating the switching apparatus 7 so as to reflux the condensate obtained in the condensing apparatus 6 to the distillation column 2 or to remove the condensate to the outside of the reactor A1 without the control of the heating temperature of the reactor A1.

The condensate which is removed from the reactor A1 by operating the switching apparatus 7 so as to connect the pipe 5b with the pipe 5d is fed to the liquid separation apparatus 8. The condensate can be efficiently separated into two layers of an upper layer and a lower layer by adding water to the condensate which is fed to the liquid separation apparatus 8. The amount of the water per 100 parts by volume of the condensate is not particularly limited, and is usually preferably 10 parts by volume or more, more preferably 20 parts by volume or more, from the viewpoint of efficient separation of the upper layer from the lower layer, and is preferably 300 parts by volume or less, more preferably 200 parts by volume or less, from the viewpoint of reduction of the amount of the resulting lower layer.

Incidentally, when water is added to the condensate, the temperature of the condensate is preferably 0°C to 50°C, more preferably 0°C to 40°C, and furthermore preferably 0°C to 30°C, from the viewpoint of efficient separation of the condensate into two layers of an upper layer and a lower layer.

The upper layer of the separated two layers mainly contains a solvent. The solvent contained in this upper layer can be effectively used as a solvent when a transesterification reaction of the (meth)acrylate which is used as a raw material and the alcohol corresponding to an objective (meth)acrylate is carried out.

Therefore, it is preferred that the above-mentioned upper layer is refluxed to the distillation column 2 in the present invention from the viewpoint of effective use of the solvent contained in the upper layer. When the above-mentioned upper layer is supplied to the distillation column 2, it is preferred that the upper layer is supplied to a middle stage of the distillation column 2 from the viewpoint of efficient progress of the transesterification reaction of the (meth)acrylate which is used as a raw material and the alcohol. Incidentally, the middle stage of the distillation column 2 means a central stage of a multistage distillation column as described above. When the distillation column 2 is, for example, a distillation column having a number of theoretical stages of 15, 6 to 9 stages of the distillation column correspond to the middle stage of the distillation column.

In addition, the fractionating capacity of the distillation column 2 can be enhanced by refluxing a part of the condensate to the upper part, preferably the top of the distillation column 2 through the pipe 5d, the switching apparatus 7 and the pipe 5c. The amount of the condensate which is refluxed to the upper part of the distillation column 2 is not particularly limited, and is preferably 20 to 95% by mass or so, more preferably 50 to 90% by mass or so of the total amount of the condensate from the viewpoint of enhancement in fractionating capacity of the distillation column 2 and increase in reaction rate.

After a part of the condensate of vapor which is taken out from the upper part of the distillation column 2 is refluxed to the upper part of the distillation column 2, it is preferred that water is added to the remaining condensate in the same manner as described above, to separate into two layers of an upper layer and a lower layer. The upper layer of the separated two layers mainly contains a solvent. Therefore, the upper layer can be effectively used as a reaction solvent which is used in the transesterification reaction of the (meth)acrylate which is used as a raw material and the alcohol corresponding to an objective (meth)acrylate in the same manner as described above. At that time, it is preferred that the above-mentioned upper layer is supplied to a middle stage of the distillation column 2 in the same manner as described above from the viewpoint of efficient progress of the transesterification reaction of the (meth)acrylate which is used as a raw material and the alcohol.

On the other hand, the above-mentioned lower layer mainly contains a by-product alcohol and water, and moreover contains a solvent and the (meth)acrylate which is used as a raw material in a content of 1 to 10% by mass or so, respectively.

When the above-mentioned lower layer is distilled, the solvent and the (meth)acrylate which is used as a raw material contained in the lower layer generate an azeotropic mixture together with the by-product alcohol. Therefore, the (meth)acrylate can be removed from the lower layer as an azeotropic mixture.

The condensate which is removed from the reactor A1 is fed to the liquid separation apparatus 8, and water is added to the condensate to separate the condensate into two layers of an upper layer and a lower layer. The lower layer of the separated two layers is fed to the distillation apparatus B3 having the distillation column 4 through the pipe 5f which connects the lower part of the liquid separation apparatus 8 with the distillation apparatus B3. The lower layer fed to the distillation apparatus B3 is distilled in the distillation apparatus B3. When this lower layer is distilled, firstly vapor containing the (meth)acrylate which is used as a raw material, the by-product alcohol and the solvent is generated by controlling the temperature at the top of the distillation column 4 to a predetermined temperature, for example, 64°C or lower in the case where the (meth)acrylate which is used as a raw material is methyl (meth)acrylate, and then vapor containing the by-product alcohol which is generated as a major component by the transesterification can be generated by controlling the temperature of the lower layer to a predetermined temperature, for example, 64°C to 66°C in the case where the (meth)acrylate which is used as a raw material is methyl (meth)acrylate. Each of the generated vapors can be taken out from the upper part of the distillation column 4.

As described above, the above-mentioned lower layer can be distilled by using the distillation apparatus B3 having the distillation column 4. The distillation apparatus B3 can be any one of a batch type distillation apparatus and a flow type distillation apparatus. When the above-mentioned lower layer is distilled, the pressure can be any of atmospheric pressure and reduced pressure. The number of theoretical stages of the distillation column 4, which is used in distillation is preferably 5 or more, more preferably 7 or more and furthermore preferably 10 or more, from the viewpoint of efficient formation of an azeotropic mixture of a solvent, the (meth)acrylate which is used as a raw material and a by-product alcohol, and is preferably 100 or less, more preferably 70 or less and furthermore preferably 50 or less, from the viewpoint of improvement in economic efficiency. In addition, when the above-mentioned lower layer is distilled, the distillation temperature is preferably 20°C or higher, more preferably 40°C or higher and furthermore preferably 50°C or higher, and is preferably 120°C or lower, more preferably 110°C or lower and furthermore preferably 105°C or lower, from the viewpoint of prevention of polymerization of the (meth)acrylate which is used as a raw material.

Since the vapor containing the (meth)acrylate which is used as a raw material, the by-product alcohol and the solvent contains the solvent, the (meth)acrylate which is used as a raw material and the by-product alcohol, it is preferred that the vapor is condensed, and the resulting condensate is used as a raw material which is used when the transesterification reaction of the (meth)acrylate which is used as a raw material and an alcohol corresponding to an objective (meth)acrylate is carried out, from the viewpoint of reduction of the amount of a waste. In addition, it is preferred that the above-mentioned vapor is condensed, the resulting condensate is distilled to separate the condensate from a by-product alcohol and water, and then the condensate is used as a raw material which is used when the transesterification reaction of the (meth)acrylate which is used as a raw material and an alcohol corresponding to an objective (meth)acrylate is carried out, from the viewpoint of effective use of the condensate without the contamination of the by-product alcohol and water. The separated by-product alcohol and water can be used, for example, as water which is added to the condensate which is removed from the reactor A1. In addition, the by-product alcohol can be easily separated from water, for example, by distillation purification and the like. Since the by-product alcohol which is separated from water which is contained in the residue contains substantially little impurities other than water, the by-product alcohol can be used, for example, as an industrial raw material, a solvent and the like, as well as an alcohol which is usually used.

When the transesterification reaction of the (meth)acrylate which is used as a raw material and the alcohol is carried out, the reaction temperature is preferably 70°C or higher and more preferably 75°C or higher, from the viewpoint of increase in reaction rate, and is preferably 140°C or lower, more preferably 130°C or lower and furthermore preferably 120°C or lower, from the viewpoint of prevention of polymerization of the (meth)acrylate which is used as a raw material and an objective (meth)acrylate.

The atmosphere where the transesterification reaction of the (meth)acrylate which is used as a raw material and the alcohol is carried out is preferably an atmosphere containing oxygen from the viewpoint of prevention of polymerization of the (meth)acrylate which is used as a raw material and an objective (meth)acrylate, and is more preferably a gas having an oxygen concentration of from 5 % by volume to an atmospheric concentration from the viewpoint of improvement in safety. The pressure of the atmosphere can be usually atmospheric pressure, and the pressure can be increased or reduced. For example, when the pressure of the atmosphere is reduced, there is an advantage such that a side reaction can be inhibited since the reflux temperature can be lowered.

In accordance with the progress of the transesterification reaction of the (meth)acrylate which is used as a raw material and an alcohol, generating rate of a by-product alcohol is lowered, and the transesterification reaction can be terminated at the time when a predetermined reaction rate is achieved. The (meth)acrylate which is used as a raw material in the reactor A1 comes to disappear due to the azeotrope with a solvent. When the (meth)acrylate which is used as a raw material disappears in the reactor A1, the temperature at the top of the distillation column 2 finally attains to a boiling point of the solvent. Therefore, the disappearance of the (meth)acrylate which is used as a raw material can be confirmed by the temperature at the top of the distillation column 2. For example, the transesterification reaction of the (meth)acrylate which is used as a raw material and an alcohol can be terminated at the time when the temperature at the top of the distillation column 2 attains to a temperature about 5°C higher than the azeotropic temperature of the solvent and a by-product alcohol. The end point of the transesterification reaction of the (meth)acrylate which is used as a raw material and an alcohol can be confirmed, for example, by means of gas chromatography, liquid chromatography and the like.

When the transesterification reaction of the (meth)acrylate which is used as a raw material and an alcohol is carried out, the reaction time cannot be absolutely determined because the reaction time differs depending on the amounts of the (meth)acrylate which is used as a raw material and the alcohol, a reaction temperature and the like. Therefore, the reaction time is usually adjusted so that an intended reaction rate is achieved.

As described above, an objective (meth)acrylate can be obtained by carrying out the transesterification reaction of the (meth)acrylate which is used as a raw material and an alcohol.

After the transesterification reaction of the (meth)acrylate which is used as a raw material and an alcohol corresponding to an objective (meth)acrylate is carried out, the resulting reaction mixture is heated to take out a vapor containing an unreacted (meth)acrylate which is used as a raw material from the upper part of the distillation column 2. According to the present invention, after the transesterification reaction is carried out as mentioned above, a vapor containing an unreacted (meth)acrylate which is used as a raw material can be taken out from the upper part of the distillation column 2 by heating the reaction mixture included in the reactor A1. When the above-mentioned procedures are employed, the (meth)acrylate which is used as a raw material can be efficiently collected from the reactor A1.

After the transesterification reaction of the (meth)acrylate which is used as a raw material and an alcohol, when the reaction mixture included in the reactor A1 is heated, the temperature cannot be absolutely determined because the temperature differs depending on the kind of a solvent used. The temperature is usually controlled to a temperature when a transesterification reaction of the (meth)acrylate which is used as a raw material and an alcohol is carried out, preferably 70°C or higher and more preferably 75°C or higher, and is preferably 140°C or lower, more preferably 130°C or lower and furthermore preferably 120°C or lower, from the viewpoint of prevention of polymerization of the (meth)acrylate which is used as a raw material and an objective (meth)acrylate. When the reaction mixture included in the reactor A1 is heated, the pressure in the reactor A1 can be controlled to atmospheric pressure. Therefore, there is no necessity to use an apparatus for pressurizing or reducing the pressure. As occasion demands, the pressure can be increased to a pressure higher than the atmospheric pressure, or reduced to a pressure lower than the atmospheric pressure.

When the reaction mixture in the reactor A1 is heated, vapor is generated. The vapor can be taken out from the upper part, preferably the top of the reactor A1, and the vapor can be cooled to collect as a condensate.

When the vapor which is generated by heating the reaction mixture in the reactor A1 is taken out from the reactor A1, the vapor contains a vapor of a solvent. Therefore, the solvent is taken out from the reactor A1 together with the (meth)acrylate which is used as a raw material, and thereby the amount of the solvent remaining in the reactor A1 decreases. Accordingly, in order to efficiently carry out the azeotrope of the (meth)acrylate which is used as a raw material and a solvent, the solvent can be added to the reactor A1 as occasion demands. The amount of solvent added is not particularly limited, and can be controlled to an amount which enables to carry out the azeotrope of the (meth)acrylate which is used as a raw material and the solvent.

The end point of the azeotrope of the (meth)acrylate which is used as a raw material and the solvent is not particularly limited. In accordance with the decrease of the amount of the (meth)acrylate which is used as a raw material, the azeotropic temperature of the azeotrope approaches to the boiling point of the solvent. Therefore, it is preferred to carry out the azeotrope until the temperature attains to the boiling point of the above-mentioned solvent from the viewpoint of efficient collection of the (meth)acrylate which is used as a raw material.

The condensate collected in the above mainly contains the (meth)acrylate which is used as a raw material, a solvent and a by-product alcohol generated by transesterification. The amount of the by-product alcohol is very smaller than the amount of the collected (meth)acrylate which is used as a raw material and the amount of the solvent. Therefore, the condensate can be effectively used as a (meth)acrylate which is used as a raw material when a transesterification reaction is newly carried out.

An objective (meth)acrylate exists in the reaction mixture in the reactor A1. The objective (meth)acrylate can be collected, for example, by taking out the reaction mixture from the lower part of the reactor A1. The reaction mixture contains a solvent which is used when a transesterification reaction is carried out, a slight amount of an unreacted (meth)acrylate and a by-product alcohol other than an objective (meth)acrylate. Therefore, the objective (meth)acrylate can be collected from the reaction mixture by removing these components from the reaction mixture as occasion demands. The removal of the unreacted (meth)acrylate, the solvent and the by-product alcohol from the reaction mixture can be easily carried out, for example, by distillation, extraction and the like.

As described above, after transesterification is carried out to prepare an objective (meth)acrylate by using the system for producing a (meth)acrylate shown in Fig. 1, the (meth)acrylate which is used as a raw material, a solvent, a by-product alcohol and the like, remaining in the reaction system can be efficiently collected and reused, and an objective (meth)acrylate can be efficiently prepared.

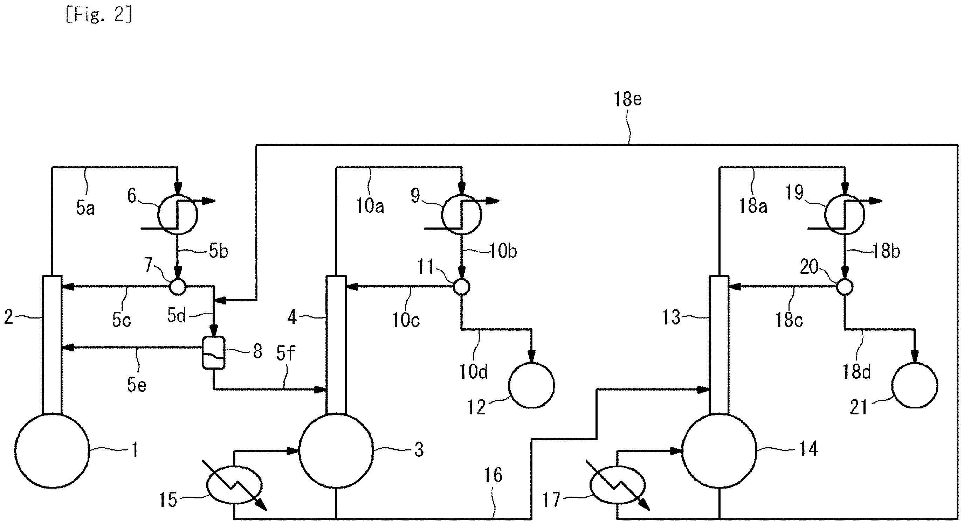

Fig.2 is a schematic drawing showing another embodiment of a system for producing a (meth)acrylate according to the present invention.

The system for producing a (meth)acrylate shown in Fig. 2 has a reactor A1 having a distillation column 2, a distillation apparatus B3 having a distillation column 4 and a distillation apparatus C14 having a distillation column 13.

The reactor A1 having the distillation column 2 shown in Fig. 2 can be the same as the reactor A1 having the distillation column 2 shown in Fig. 1.

In addition, the distillation apparatus B3 having the distillation column 4 shown in Fig. 2 can be the same as the distillation apparatus B3 having the distillation column 4 shown in Fig. 1. Incidentally, the distillation apparatus B3 can be provided with, for example, a heating apparatus 15 such as a reboiler for heating contents in the distillation apparatus B3 as occasion demands.

The condensate removed from the reactor A1 is fed to the separation apparatus 8, and water is added to the condensate, to separate the condensate into two layers of an upper layer and a lower layer in the same manner as in the above-mentioned embodiment of the system for producing a (meth)acrylate shown in Fig. 1. The lower layer of the separated two layers is fed to the distillation apparatus B3 having the distillation column 4 through a pipe 5f, and distilled.

It is preferred that the position of the distillation column 4 where the above-mentioned lower layer is fed to the distillation column 4 is a middle stage of the distillation column 4 from the viewpoint of efficient separation of a solvent contained in the lower layer from a by-product alcohol and water generated as a by-product. Incidentally, the middle stage of the distillation column 4 means a central stage of the multistage distillation column. When the distillation column 4 is, for example, a distillation column having a number of theoretical stages of 15, 6 to 9 stages of the distillation column correspond to the middle stage of the distillation column.

When the above-mentioned lower layer is distilled, firstly vapor containing the (meth)acrylate which is used as a raw material, a by-product alcohol and a solvent is generated by controlling the temperature at the top of the distillation column 4 to a predetermined temperature, for example, 64°C or lower in the case where the (meth)acrylate which is used as a raw material is methyl (meth)acrylate, and then vapor containing the by-product alcohol which is generated by the transesterification reaction as a major component can be generated by controlling the temperature of the lower layer to a predetermined temperature, for example, 64°C to 66°C in the case where the (meth)acrylate which is used as a raw material is methyl (meth)acrylate. Each of the generated vapors can be taken out from the upper part of the distillation column 4.

When the above-mentioned lower layer is distilled, the pressure can be any of atmospheric pressure and reduced pressure. The number of theoretical stages of the distillation column 4 which is used in distillation is preferably 5 or more, more preferably 7 or more and furthermore preferably 10 or more, from the viewpoint of efficient formation of an azeotropic mixture of a solvent, the (meth)acrylate which is used as a raw material and a by-product alcohol, and is preferably 100 or less, more preferably 70 or less and furthermore preferably 50 or less, from the viewpoint of improvement in economic efficiency. In addition, when the above-mentioned lower layer is distilled, the distillation temperature is preferably 20°C or higher, more preferably 40°C or higher and furthermore preferably 50°C or higher, and is preferably 120°C or lower, more preferably 110°C or lower and furthermore preferably 105°C or lower, from the viewpoint of prevention of polymerization of the (meth)acrylate which is used as a raw material.

The vapor containing the (meth)acrylate which is used as a raw material, a by-product alcohol and a solvent contains the solvent, the (meth)acrylate which is used as a raw material and the by-product alcohol. Therefore, the upper part, preferably the top of the distillation column 4 of the distillation apparatus B3 is connected with a condensing apparatus 9 through a pipe 10a. The condensing apparatus 9 is connected with a switching apparatus 11 through a pipe 10b, and the switching apparatus 11 is connected with the upper part, preferably the top of the distillation column 4 through a pipe 10c in order to reflux a part of the condensate obtained in the condensing apparatus 9 to the upper part, preferably the top of the distillation column 4.

The condensing apparatus 9 is connected with the switching apparatus 11 through the pipe 10b, and the switching apparatus 11 is connected with a collecting unit 12 through a pipe 10d in order to collect the condensate remaining in the condensing apparatus 9. The condensate obtained by condensing the above-mentioned vapor in the condensing apparatus 9 mainly contains a solvent, the (meth)acrylate which is used as a raw material and a by-product alcohol. Therefore, the above-mentioned condensate can be effectively used by collecting the condensate in the collecting unit 12, and returning the condensate collected in the collecting unit 12 to the reactor A1.

A liquid mainly containing water and the by-product alcohol exists in the lower part or the bottom of the distillation apparatus B3 having the distillation column 4. The liquid is fed to the distillation apparatus C14 having the distillation column 13 through a pipe 16 which is connected with the distillation apparatus B3 at the lower part or the bottom of the distillation apparatus B3.

The distillation apparatus C14 having the distillation column 13 can be the same as the distillation apparatus B3 having the distillation column 4 shown in Fig. 1. Incidentally, the distillation apparatus C14 can be provided with, for example, a heating apparatus 17 such as a reboiler in order to heat the contents in the distillation apparatus C14 as occasion demands.

It is preferred that the position of the distillation column 13 where the above-mentioned liquid is fed is a middle stage of the distillation column 13 from the viewpoint of efficient separation of water contained in the liquid from a by-product alcohol. Incidentally, as described above, the middle stage of the distillation column 13 means a stage positioned at around the center of the multistage distillation column. When the distillation column 13 is, for example, a distillation column having a number of theoretical stages of 15, 6 to 9 stages of the distillation column correspond to the middle stage of the distillation column.

When the above-mentioned liquid is distilled, vapor containing a by-product alcohol and water vapor can be generated, and the generated vapor can be taken out from the top of the distillation column 13 by controlling the temperature of the top of the distillation column 13 to a predetermined temperature, for example, 64°C to 66°C in the case where the by-product alcohol is methanol.

The distillation apparatus C14 can be any one of a batch type distillation apparatus and a flow type distillation apparatus. A preferred distillation apparatus C14 having the distillation column 13 includes, for example, a packed column, a tray type distillation column and the like, and the present invention is not limited only to those exemplified ones. A filler used in the packed column includes, for example, Helipack, McMahon, Cascade Mini Ring and the like, and the present invention is not limited only to those exemplified ones. The tray type distillation column includes, for example, a packed distillation column, an Oldershaw distilling column, a Lift Tray distillation column and the like, and the present invention is not limited only to those exemplified ones.

When the above-mentioned liquid is distilled, the pressure can be any of atmospheric pressure and reduced pressure. The number of theoretical stages of the distillation column 13 which is used in distillation is preferably 5 or more, more preferably 7 or more and furthermore preferably 10 or more, from the viewpoint of efficient formation of an azeotropic mixture of a by-product alcohol and water, and is preferably 100 or less, more preferably 70 or less and furthermore preferably 50 or less, from the viewpoint of improvement in economic efficiency. In addition, when the above-mentioned liquid is distilled, the distillation temperature is preferably 20°C or higher, more preferably 40°C or higher and furthermore preferably 50°C or higher, and is preferably 120°C or lower, more preferably 110°C or lower and furthermore preferably 105°C or lower, from the viewpoint of improvement in chemical stability of an objective (meth)acrylate.

In order to separate the by-product alcohol contained in the above-mentioned liquid from water, the upper part, preferably the top of the distillation column 13 of the distillation apparatus C14 is connected with a condensing apparatus 19 through a pipe 18a. In order to reflux a part of the condensate obtained in the condensing apparatus 19 to the upper part, preferably the top of the distillation column 13, the condensing apparatus 19 is connected with a switching apparatus 20 through a pipe 18b, and the switching apparatus 20 is connected with the upper part, preferably the top of the distillation column 13 through a pipe 18c by operating the switching apparatus 20.

The switching apparatus 20 includes, for example, a switching valve, a reflux distributor, a solenoid valve and the like, and the present invention is not limited only to those exemplified ones.

The condensate obtained by condensing the above-mentioned vapor in the condensing apparatus 19 mainly contains a by-product alcohol. When this by-product alcohol is collected in a collecting unit 21, the condensing apparatus 19 is connected with the switching apparatus 20 through the pipe 18b, and the switching apparatus 20 is connected with the collecting unit 21 through a pipe 18d by operating the switching apparatus 20. The by-product alcohol collected in the collecting unit 21 can be effectively used, for example, as a fuel, a raw material for preparing various organic compounds, and the like. The collecting unit 21 can be a so-called receiver.

In the lower part or the bottom of the distillation apparatus C14 having the distillation column 13, water is mainly exists. The water can be effectively used in the liquid separation apparatus 8 of the reactor A1 by connecting the lower part or the bottom of the distillation apparatus C14 with the pipe 5d through a pipe 18e. The pipe 18e can be easily connected with the pipe 5d by means of, for example, a three-way cock (not shown in the figure) and the like.

As described above, when the system for producing a (meth)acrylate shown in Fig. 2 is employed, after the preparation of a (meth)acrylate by the transesterification, a (meth)acrylate which is used as a raw material, a solvent, an alcohol generated as a by-product and the like which are remaining in a reaction system can be efficiently collected and reused, and an objective (meth)acrylate can be efficiently prepared.

Incidentally, the (meth)acrylate which is used as a raw material is usually malodorous. Therefore, when the (meth)acrylate is exposed to the air, strong bad smell is emitted to the air. Therefore, it has been desired that the (meth)acrylate is not exposed to the air, or that the amount of the (meth)acrylate exposed to the air is reduced as much as possible.

To the contrary, according to the present invention, since a (meth)acrylate used as a raw material remains in a reaction mixture after transesterification, and the remaining (meth)acrylate can be gently and easily removed from a reaction mixture by the azeotrope of the (meth)acrylate which can be used as a raw material and a solvent under atmospheric pressure, the amount of the (meth)acrylate which is used as a raw material, exhausted to the air can be reduced as much as possible.

Therefore, it can be said that the system for producing a (meth)acrylate of the present invention is an environment-friendly system which considers environmental impact.

Incidentally, an exhaust port of the reactor A1 can be equipped with an alkali scrubber and the like as occasion demands in order to adsorb the (meth)acrylate which is used as a raw material, and inhibit the exhaustion of the (meth)acrylate to the air.

In addition, it is sometimes desired that a (meth)acrylate which is used as a raw material is not is contaminated in an objective (meth)acrylate depending on the kind, use and the like of the objective (meth)acrylate. The system for producing a (meth)acrylate of the present invention is also useful as a system which avoids the contamination of a (meth)acrylate which is used as a raw material in an objective (meth)acrylate.

Furthermore, when the system for producing a (meth)acrylate of the present invention is employed, a by-product alcohol can be collected in a high purity, a by-product (meth)acrylate generated by transesterification can be effectively used, and by-product water also can be effectively used. Therefore, the system for producing a (meth)acrylate of the present invention is a system (apparatus) which is excellent in industrial productivity of a (meth)acrylate.

In addition, since the system for producing a (meth)acrylate of the present invention is a so-called closed system, the amount of a by-product exhausted to the outside of the reaction system can be reduced, and a yield of an objective (meth)acrylate can be improved.

The (meth)acrylate prepared by using the system for producing a (meth)acrylate of the present invention is useful, for example, as a raw material for a (meth)acrylic resin, a surfactant, an adhesive, a paint, and the like.

### EXAMPLES

Next, the present invention will be more specifically described based on working examples. However, the present invention will not be limited only to those examples.

In the following examples, the yield of an objective (meth)acrylate was determined based on the ratio of the actually resulting amount of the objective (meth)acrylate to the theoretically resulting amount of the objective (meth)acrylate.

In addition, each amount of a raw material alcohol, an objective (meth)acrylate obtained from the raw material alcohol, a solvent and a (meth)acrylate which was used as a raw material in the reaction mixture obtained in the following examples was determined based on an area percentage in gas chromatography (hereafter referred to as GC) obtained by using a GC analyzer (manufactured by Agilent Technologies Ltd., detector: FID, column capillary: DB-1: 30m).

### Example 1

A system for producing a (meth)acrylate shown in Fig. 1 was used. As a reactor A having a distillation column, a 2-liter four-necked flask having a 20-stage Oldershaw distillation column (number of theoretical stages: 15) equipped with a condensing apparatus at its top and having a side tube, and an air intake tube was used. The flask was charged with 694 g (8.06 moles) of methyl acrylate, 552 g (6.20 moles) of N,N-dimethylaminoethanol, 1.76 g of phenothiazine, 22.1 g of dibutyltin oxide and 100 g of cyclohexane. A transesterification reaction was carried out while blowing air into the flask from the air intake tube at a flow rate of 20 mL/min. More specifically, vapor which was taken out from the top of the distillation column was condensed in the condensing apparatus, a part of the resulting condensate was refluxed to the top of the distillation column, and the remaining condensate was removed from the flask. The amount of the condensate which was removed from the top of the distillation column was adjusted to control the temperature of the top of the distillation column to an azeotropic temperature of methyl alcohol and cyclohexane of 54°C to 56°C.

The condensate which was removed from the top of the distillation column in an amount of 700 g was mixed with 200 g of water at a temperature of 20°C, and the resulting mixed solution was fed to a decanter. This mixed solution was separated into two layers of an upper layer and a lower layer. Since methyl alcohol which was contained in the condensate was extracted with water, the methyl alcohol was contained in the lower layer, and the cyclohexane which had been included in the condensate was contained in the upper layer.

The amount of the above-mentioned lower layer was 410 g. The lower layer contained 47.0% by mass (193 g) of methyl alcohol, 0.5% by mass (2 g) of cyclohexane, 3.6% by mass (15 g) of methyl acrylate and 48.9% by mass (200 g) of water.

On the other hand, the above-mentioned upper layer was effectively used by feeding the upper layer to the distillation column at the middle stage of the distillation column where a 10th stage was provided from the bottom of the distillation column.

A transesterification reaction of methyl acrylate and N,N-dimethylaminoethanol was carried out while properly adding cyclohexane to the flask so that the reaction temperature was 85°C to 102°C. When 4 hours passed from the initiation of the reaction, the transesterification reaction was terminated. After the termination of the reaction, the amount of methyl acrylate contained in the reaction mixture which was obtained by the above-mentioned transesterification reaction was 160 g.

After the termination of the reaction, a reaction mixture obtained by the above-mentioned transesterification reaction was further heated, and a reflux ratio was controlled to 10 to 15. A vapor was taken out from the top of the distillation column, and the vapor was condensed to give a condensate. At that time, cyclohexane was added to the flask so that the temperature of the reaction mixture included in the flask was maintained to 85°C to 100°C. While taking out the condensate from the top of the distillation column, the temperature at the top of the distillation column was gradually increased. The distillation was continued until the temperature at the top of the distillation column attained to a boiling point of cyclohexane of 80°C.

As a result, the content of methyl acrylate in the reaction mixture included in the flask was at most 0.1% by mass. The condensate was taken out from the top of the distillation column, and collected. The amount of the collected condensate was 490 g, and this condensate contained 5 g of methanol, 145 g of methyl acrylate and 340 g of cyclohexane. This condensate contained most of the methyl acrylate which was used in an excessive amount. This collected condensate could be used when a transesterification reaction of methyl acrylate and N,N-dimethylaminoethanol was carried out. From this fact, it was confirmed that loss of methyl acrylate could be prevented by using the above-mentioned condensate when a transesterification reaction of methyl acrylate and N,N-dimethylaminoethanol was carried out.

As a result of the preparation of N,N-dimethylaminoethyl acrylate as described above, the yield of N,N-dimethylaminoethyl acrylate was 852 g (yield on the basis of N,N-dimethylaminoethanol: 96% by mass, yield on the basis of the amount of methyl acrylate used: 73.8% by mass, yield in the case where methyl acrylate which was collected by the azeotrope with cyclohexane after the termination of the reaction was reused: 93.3% by mass).

From the above results, when a reactor A is used, it can be seen that a condensate taken out from the upper part of the distillation column can be efficiently used by mixing the condensate with water, separating the resulting mixed solution into two layers, and feeding the resulting upper layer to the distillation column, that a condensate obtained by heating a reaction mixture prepared by transesterification, and collecting from the top of the distillation column can be effectively used in a new transesterification reaction, and that an objective N,N-dimethylaminoethyl acrylate can be prepared in a high yield.

Next, as a distillation apparatus B, a 1-liter four-necked flask having a 10-stage Oldershaw distillation column (number of theoretical stages: 7) equipped with a condensing apparatus at its top and having a side tube, and an air intake tube was used. The flask was charged with the lower layer obtained in the above.

A distillation was carried out while refluxing in a reflux ratio of 10 to 15. When the temperature at the top of the distillation column was 64°C or lower, a fraction A was collected. As a result, the amount of the collected fraction A was 40 g, and this fraction A was composed of 62.1% by mass (25 g) of methyl alcohol, 32.5% by mass (13 g) of methyl acrylate, 5.3% by mass (2 g) of cyclohexane and 0.1% by mass of water. This fraction A could be used when a transesterification reaction of methyl acrylate and N,N-dimethylaminoethanol was carried out.

The above-mentioned distillation was continued in a reflux ratio of 5 to 10. When the temperature at the top of the distillation column was 64°C to 66°C, a fraction B was collected. As a result, the amount of the collected fraction B was 167 g. This fraction B was composed of 99.9% by mass of methyl alcohol and 0.1% by mass of water, and it was not confirmed that methyl acrylate was included.

The above-mentioned fraction B was analyzed by the GC. As a result, no component other than methyl alcohol and a slight amount of water was detected. Therefore, it was confirmed that the fraction B could be effectively used as an industrial raw material, a solvent and the like. In addition, since methyl acrylate was not detected in the fraction B by the GC, it was confirmed that loss of methyl acrylate was little, and therefore a yield of N,N-dimethylaminoethyl acrylate was not adversely affected.

In addition, 203 g of the residual solution in the flask was composed of 3% by mass of methyl alcohol and 97% by mass of water, and a component other than these compounds was not detected by the GC. Since the residual solution mainly contained water, the residual solution could be used as water which was used in mixing a condensate with water in Example 1.

From the above results, it can be seen that there can be effectively used a lower layer obtained by mixing a condensate which was taken out from the top of the distillation column of the above-mentioned reactor A with water, and separating the resulting mixed solution into two layers.

### Example 2

A reactor A which was the same kind as that used in Example 1 was used. The flask was charged with 517 g (6.00 moles) of methyl acrylate, 651 g (5.00 moles) of n-octyl alcohol, 1.76 g of phenothiazine, 0.72 g of tetramethyltitanium and 117 g of n-hexane. A transesterification reaction was carried out while blowing air into the flask from the air intake tube at a flow rate of 20 mL/min. More specifically, a vapor which was taken out from the top of the distillation column was condensed, a part of the resulting condensate was refluxed to the top, and the remaining condensate was removed from the flask. The temperature at the top of the distillation column was controlled to an azeotropic temperature of methyl alcohol and n-hexane of 48°C to 50°C by adjusting the amount of the condensate removed from the flask.

The condensate which was removed from the top of the distillation column in an amount of 549 g was mixed with 100 g of water at a temperature of 20°C, and the resulting mixed solution was introduced to a decanter. This mixed solution was separated into two layers of an upper layer and a lower layer. Since methyl alcohol contained in the condensate was extracted with water, the methyl alcohol was contained in the lower layer, and the n-hexane included in the condensate was contained in the upper layer.

The amount of the above-mentioned lower layer was 257 g, and the lower layer contained 56.4% by mass (145 g) of methyl alcohol, 1.6% by mass (4 g) of n-hexane, 3.1% by mass (8 g) of methyl acrylate and 38.9% by mass (100 g) of water.

On the other hand, the above-mentioned upper layer was effectively used by feeding the upper layer to the distillation column at the middle stage of the distillation column where a 10th stage was provided from the bottom of the distillation column.

The reaction temperature in the transesterification reaction of methyl acrylate and n-octyl alcohol was 90°C to 110°C. When 8 hours passed from the initiation of the reaction, the transesterification reaction was terminated. After the termination of the reaction, the amount of methyl acrylate contained in the reaction mixture which was obtained by the above-mentioned transesterification reaction was 86 g.

After the termination of the reaction, a reaction mixture obtained by the above-mentioned transesterification reaction was further heated in a reflux ratio of 10 to 15. A vapor was taken out from the top of the distillation column, and the vapor was condensed to collect a condensate. At that time, n-hexane was added to the flask so that the temperature of the reaction mixture included in the flask was maintained to 90°C to 110°C. While taking out the condensate from the top of the distillation column, the temperature at the top of the distillation column was gradually increased. The distillation was continued until the temperature at the top of the distillation column attained to a boiling point of n-hexane of 68°C.

As a result, the content of methyl acrylate in the reaction mixture included in the flask was at most 0.2% by mass. In addition, the amount of the condensate taken out from the top of the distillation column and collected was 392 g. This condensate contained 3.8% by mass (15 g) of methanol, 18.6% by mass (73 g) of methyl acrylate and 77.6% by mass (304 g) of n-hexane. This condensate contained most of the methyl acrylate used in an excessive amount. This collected condensate could be used when a transesterification reaction of methyl acrylate and n-octyl alcohol was carried out. From this fact, it was confirmed that loss of methyl acrylate could be prevented by using the above-mentioned condensate when a transesterification reaction of methyl acrylate and n-octyl alcohol is carried out.

As a result of the preparation of n-octyl acrylate as described above, the yield of n-octyl acrylate was 912 g (yield on the basis of n-octyl acrylate: 99.0% by mass; yield on the basis of the amount of methyl acrylate used: 82.5% by mass; yield in the case where methyl acrylate which was collected by azeotrope with n-hexane after the termination of the reaction was reused: 96.0% by mass).

Next, as a distillation apparatus B, a 2-liter four-necked flask having a 10-stage Oldershaw distillation column (number of theoretical stages: 7) equipped with a condensing apparatus at its top and having a side tube, and an air intake tube was used. The flask was charged with the lower layer obtained in the above.

A distillation was carried out while refluxing in a reflux ratio of 10 to 15. When the temperature at the top of the distillation column was 64°C or lower, a fraction A was collected. As a result, the amount of the collected fraction A was 32 g, and this fraction A was composed of 62.5% by mass (20 g) of methyl alcohol, 25.0% by mass (8 g) of methyl acrylate, 12.5% by mass (4 g) of n-hexane and 0.1% by mass of water. This fraction A could be used when a transesterification reaction of methyl acrylate and n-octyl alcohol was carried out.

The above-mentioned distillation was continued in a reflux ratio of 5 to 10. When the temperature of the top of the distillation column was 64°C to 66°C, a fraction B was collected. As a result, the amount of the collected fraction B was 122 g. This fraction B contained 99.9% by mass (122 g) of methyl alcohol and 0.1% by mass of water, and it was not confirmed that methyl acrylate was included.

Since the above-mentioned fraction B did not contain a component other than methyl alcohol and a slight amount of water, it was confirmed that the fraction B could be effectively used as an industrial raw material, a solvent and the like. In addition, since methyl acrylate was not detected in the fraction B by the GC, it was confirmed that loss of methyl acrylate was little, and therefore a yield of n-octyl acrylate was not adversely affected.

In addition, 103 g of the residual solution in the flask was composed of 3% by mass (3 g) of methyl alcohol and 97% by mass (100 g) of water, and a component other than these compounds was not detected by the GC. Since the residual solution mainly contained water, the residual solution could be used as water which was used in mixing a condensate with water.

### Example 3

A reactor A which was the same kind as that used in Example 1 was used. The flask was charged with 430 g (5.0 moles) of methyl acrylate, 675 g (7.5 moles) of 1,4-butanediol, 0.72 g of phenothiazine, 3.6 g of dioctyltin oxide and 100 g of cyclohexane. A transesterification reaction was carried out while blowing air into the flask from the air intake tube at a flow rate of 20 mL/min. More specifically, vapor which was taken out from the top of the distillation column was condensed, a part of the resulting condensate was refluxed to the top, and the remaining condensate was removed from the reactor A. The temperature at the top of the distillation column was controlled to an azeotropic temperature of methyl alcohol and cyclohexane of 54°C to 56°C by adjusting the amount of the condensate removed from the flask.

The condensate which was removed from the top of the distillation column in an amount of 490 g was mixed with 140 g of water at a temperature of 20°C, and the resulting mixed solution was introduced to a decanter. This mixed solution was separated into two layers of an upper layer and a lower layer. Since methyl alcohol contained in the condensate was extracted with water, the methyl alcohol was contained in the lower layer, and the cyclohexane included in the condensate was contained in the upper layer.

The amount of the above-mentioned lower layer was 303 g, and the lower layer contained 48.7% by mass (148 g) of methyl alcohol, 1.6% by mass (5 g) of cyclohexane, 3.3% by mass (10 g) of methyl acrylate and 46.4% by mass (140 g) of water.

On the other hand, the above-mentioned upper layer was effectively used by feeding the upper layer to the distillation column at the middle stage of the distillation column where a 10th stage was provided from the bottom of the distillation column.

A transesterification reaction of methyl acrylate and 1,4-butanediol was carried out while properly adding cyclohexane to the flask so that the reaction temperature was 85°C to 100°C. When 8 hours passed from the initiation of the reaction, the transesterification reaction was terminated. After the termination of the reaction, the amount of methyl acrylate contained in the reaction mixture which was obtained by the above-mentioned transesterification reaction was 20 g.

After the termination of the reaction, a reaction mixture obtained by the above-mentioned transesterification reaction was further heated in a reflux ratio of 10 to 15. A vapor was taken out from the top of the distillation column, and the vapor was condensed to collect a condensate. At that time, cyclohexane was added to the flask so that the temperature of the reaction mixture included in the flask was maintained to 85°C to 100°C. While taking out the condensate from the top of the distillation column, the temperature at the top of the distillation column gradually increased. The distillation was continued until the temperature at the top of the distillation column attained to a boiling point of cyclohexane of 80°C.

As a result, the content of methyl acrylate in the reaction mixture included in the flask was at most 0.2% by mass. In addition, the amount of the condensate taken out from the top of the distillation column and collected was 75 g. This condensate contained 5 g of methanol, 10 g of methyl acrylate and 60 g of cyclohexane. This condensate contained most of the methyl acrylate used in an excessive amount. This collected condensate could be used when a transesterification reaction of methyl acrylate and 1,4-butanediol was carried out. From this fact, it was confirmed that loss of methyl acrylate could be prevented by using the above-mentioned condensate when a transesterification reaction of methyl acrylate and 1,4-butanediol is carried out.

As a result of the preparation of 4-hydroxybutyl acrylate as described above, the obtained reaction mixture contained 496 g of 4-hydroxybutyl acrylate, 131 g of 1,4-butanediol diacrylate and 305 g of 1,4-butanediol. The 4-hydroxybutyl acrylate contained in this reaction mixture could be separated by extracting the reaction mixture, distilling the reaction mixture or adsorbing with an adsorption column.

Next, as a distillation apparatus B, a 1-liter four-necked flask having a 10-stage Oldershaw distillation column (number of theoretical stages: 7) equipped with a condensing apparatus at its top and having a side tube, and an air intake tube was used. The flask was charged with the lower layer obtained in the above.

A distillation was carried out while refluxing in a reflux ratio of 10 to 15. When the temperature at the top of the distillation column was 64°C or lower, a fraction A was collected. As a result, the amount of the collected fraction A was 42 g, and this fraction A was composed of 64.3% by mass (27 g) of methyl alcohol, 23.8% by mass (10 g) of methyl acrylate, 12.5% by mass (5 g) of cyclohexane and 0.1% by mass (0.04 g) of water. This fraction A could be used when a transesterification reaction of methyl acrylate and 1,4-butanediol was carried out.

The above-mentioned distillation was continued in a reflux ratio of 5 to 10. When the temperature at the top of the distillation column was 64°C to 65°C, a fraction B was collected. As a result, the amount of the collected fraction B was 118 g. This fraction B was composed of 99.9% by mass of methyl alcohol and 0.1% by mass of water, and it was not confirmed that methyl acrylate was included.

Since the above-mentioned fraction B did not contain a component other than methyl alcohol and a slight amount of water, it was confirmed that the fraction B could be effectively used as an industrial raw material, a solvent and the like. In addition, since methyl acrylate was not detected in the fraction B by the GC, it was confirmed that loss of methyl acrylate was little, and therefore a yield of 4-hydroxybutyl acrylate was not adversely affected.

In addition, 142 g of the residual solution in the flask was composed of 3% by mass of methyl alcohol and 97% by mass of water, and a component other than these compounds was not detected by the GC. Since the residual solution mainly contained water, the residual solution could be used as water which was used in mixing a condensate with water.

### Example 4

A transesterification reaction was carried out in the same manner as in Example 1 except that the same reactor A as used in Example 1 was charged with 780.9 g (7.80 moles) of methyl methacrylate, 372.4 g (6.00 moles) of N,N-dimethylaminoethanol and 1.89 g of phenothiazine. After the termination of the reaction, the amount of methyl methacrylate contained in the reaction mixture which was obtained by the transesterification reaction was 178 g.

After cyclohexane was added to the flask, and 175 g of unreacted methyl methacrylate was collected, the content of methyl methacrylate in the reaction mixture included in the flask was determined. As a result, the content of methyl methacrylate was at most 0.1% by mass.

Next, the lower layer of the decanter was distilled in the same manner as in Example 1, and 5 g of a fraction A was collected. This fraction A was composed of 86.9% by mass of methyl alcohol, 13.0% by mass of cyclohexane and 0.1% by mass of water. This fraction A could be used when a transesterification reaction of methyl methacrylate and N,N-dimethylaminoethanol was carried out. Subsequently, a fraction B was collected in the same manner as in Example 1, and its components were examined. As a result, this fraction B was composed of 99.9% by mass of methyl alcohol and 0.1% by mass of water.

### Example 5

A transesterification reaction was carried out in the same manner as in Example 1 except that the same reactor A as used in Example 1 was charged with 1041.2 g (10.4 moles) of methyl methacrylate, 248.3 g (4.00 moles) of ethylene glycol, 1.59 g of phenothiazine and 0.96 g of lithium hydroxide. After the termination of the reaction, the amount of methyl methacrylate contained in the reaction mixture which was obtained by the transesterification reaction was 235 g.

After cyclohexane was added to the flask, and 233 g of unreacted methyl methacrylate was collected, the content of methyl methacrylate in the reaction mixture included in the flask was examined. As a result, the content of methyl methacrylate was at most 0.1% by mass.

Next, the lower layer of the decanter was distilled in the same manner as in Example 1, and 5 g of a fraction A was collected. This fraction A was composed of 86.9% by mass of methyl alcohol, 13.0% by mass of cyclohexane and 0.1% by mass of water. This fraction A could be used when a transesterification reaction of methyl methacrylate and ethylene glycol was carried out. Subsequently, a fraction B was collected in the same manner as in Example 1, and its components were examined. As a result, this fraction B was composed of 99.9% by mass of methyl alcohol and 0.1% by mass of water.

### Example 6

A transesterification reaction was carried out in the same manner as in Example 1 except that 633.2 g (6.20 moles) of tetrahydrofurfuryl alcohol was used in place of N,N-dimethylaminoethanol in Example 1. After the termination of the reaction, the amount of methyl acrylate contained in the reaction mixture which was obtained by the transesterification reaction was 147 g.

After cyclohexane was added to the flask, and 146 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.3% by mass of methyl alcohol, 32.1% by mass of methyl acrylate, 5.5% by mass of cyclohexane and 0.1% by mass of water. This fraction A could be used when a transesterification reaction of methyl acrylate and tetrahydrofurfuryl alcohol was carried out. Subsequently, a fraction B was collected in the same manner as in Example 2, and its components were examined. As a result, this fraction B was composed of 99.9% by mass of methyl alcohol and 0.1% by mass of water, and it was not confirmed that methyl acrylate was included.

### Example 7

A transesterification reaction was carried out in the same manner as in Example 1 except that 805.0 g (6.00 moles) of diethylene glycol monoethyl ether was used in place of N,N-dimethylaminoethanol in Example 1. After the termination of the reaction, the amount of methyl acrylate contained in the reaction mixture which was obtained by the transesterification reaction was 140 g.

After cyclohexane was added to the flask, and 138 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.3% by mass of methyl alcohol, 32.3% by mass of methyl acrylate, 5.3% by mass of cyclohexane and 0.1% by mass of water. This fraction A could be used when a transesterification reaction of methyl acrylate and diethylene glycol monoethyl ether was carried out. Subsequently, a fraction B was collected in the same manner as in Example 2, and its components were examined. As a result, this fraction B was composed of 99.9% by mass of methyl alcohol and 0.1% by mass of water, and it was not confirmed that methyl acrylate was included.

### Example 8

A transesterification reaction was carried out in the same manner as in Example 1 except that 894.8 g (5.20 moles) of methyl acrylate, 472.7 g (4.00 moles) of 1,6- hexane diol and 3.7 g of dioctyltin oxide were used in Example 1. After the termination of the reaction, the amount of methyl acrylate contained in the reaction mixture which was obtained by the transesterification reaction was 189 g.

After cyclohexane was added to the flask, and 188 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.1% by mass of methyl alcohol, 32.4% by mass of methyl acrylate, 5.4% by mass of cyclohexane and 0.1% by mass of water. This fraction A could be used when a transesterification reaction of methyl acrylate and 1,6-hexane diol was carried out. Subsequently, a fraction B was collected in the same manner as in Example 1, and its components were examined. As a result, this fraction B was composed of 99.9% by mass of methyl alcohol and 0.1% by mass of water, and it was not confirmed that methyl acrylate was included.

### Example 9

A transesterification reaction was carried out in the same manner as in Example 1 except that 1006.7 g (11.7 moles) of methyl acrylate, 402.5 g (3.00 moles) of trimethylolpropane and 8.4 g of dioctyltin oxide were used in Example 1. After the termination of the reaction, the amount of methyl acrylate contained in the reaction mixture which was obtained by the transesterification reaction was 216 g.

After cyclohexane was added, and 214 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.1% by mass of methyl alcohol, 32.3% by mass of methyl acrylate, 5.5% by mass of cyclohexane and 0.1% by mass of water. This fraction A could be used when a transesterification reaction of methyl acrylate and trimethylolpropane was carried out. Subsequently, a fraction B was collected in the same manner as in Example 1, and its components were examined. As a result, this fraction B was composed of 99.9% by mass of methyl alcohol and 0.1% by mass of water, and it was not confirmed that methyl acrylate was included.

### Example 10

A transesterification reaction was carried out in the same manner as in Example 1 except that 559.3 g (6.5 moles) of methyl acrylate, 1041.5 g (5.00 moles) of 2-ethyl-2-methyl-1,3-dioxolane-4-methanol and 4.7 g of dioctyltin oxide were used in Example 1. After the termination of the reaction, the amount of methyl acrylate contained in the reaction mixture which was obtained by the transesterification reaction was 117 g.

After cyclohexane was added to the flask, and 116 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.5% by mass of methyl alcohol, 32.1% by mass of methyl acrylate, 5.3% by mass of cyclohexane and 0.1% by mass of water. This fraction A could be used when a transesterification reaction of methyl acrylate and 2-ethyl-2-methyl-1,3-dioxolane-4-methanol was carried out. Subsequently, a fraction B was collected in the same manner as in Example 2, and its components were examined. As a result, this fraction B was composed of 99.9% by mass of methyl alcohol and 0.1% by mass of water, and it was not confirmed that methyl acrylate was included.

### Example 11

A transesterification reaction was carried out in the same manner as in Example 1 except that 615.2 g (7.15 moles) of methyl acrylate, 892.1 g (5.50 moles) of 3-ethyl-3-oxetanylmethyl alcohol and 5.1 g of dioctyltin oxide were used in Example 1. After the termination of the reaction, the amount of methyl acrylate contained in the reaction mixture which was obtained by the transesterification reaction was 123 g.

After cyclohexane was added to the flask, and 122 g of unreacted methyl acrylate was collected, the content of methyl acrylate in the reaction mixture included in the flask was examined. As a result, the content of methyl acrylate was at most 0.1% by mass.

Next, the lower layer of the decanter was distilled in the same manner as in Example 1. As a result, a fraction A was composed of 62.3% by mass of methyl alcohol, 32.1% by mass of methyl acrylate, 5.5% by mass of cyclohexane and 0.1% by mass of water. This fraction A could be used when a transesterification reaction of methyl acrylate and 3-ethyl-3-oxetanylmethyl alcohol was carried out. Subsequently, a fraction B was collected in the same manner as in Example 2, and its components were examined. As a result, this fraction B was composed of 99.9% by mass of methyl alcohol and 0.1% by mass of water, and it was not confirmed that methyl acrylate was included.

### Example 12

A system for producing a (meth)acrylate shown in Fig. 2 was used. As a reactor A having a distillation column, a 2-liter four-necked flask having a 20-stage Oldershaw distillation column (number of theoretical stages: 15) equipped with a condensing apparatus at its top and having a side tube, and an air intake tube was used. The flask was charged with 694 g (8.06 moles) of methyl acrylate, 552 g (6.20 moles) of N,N-dimethylaminoethanol, 1.76 g of phenothiazine, 22.1 g of dibutyltin oxide and 100 g of cyclohexane. A transesterification reaction was carried out while blowing air into the flask from the air intake tube at a flow rate of 20 mL/min. More specifically, vapor which was taken out from the top of the distillation column was condensed in the condensing apparatus, a part of the resulting condensate was refluxed to the top of the distillation column, and the remaining condensate was removed from the flask. The temperature at the top of the distillation column was controlled to an azeotropic temperature of methyl alcohol and cyclohexane of 54°C to 56°C by adjusting the amount of the condensate removed.

The condensate which was removed from the top of the distillation column in an amount of 700 g was mixed with 200 g of water at a temperature of 20°C, and the resulting mixed solution was fed to a decanter. This mixed solution was separated into two layers of an upper layer and a lower layer. Since methyl alcohol contained in the condensate was extracted with water, the methyl alcohol was contained in the lower layer, and the cyclohexane included in the condensate was contained in the upper layer.

The amount of the above-mentioned lower layer was 410 g, and the lower layer contained 48.5% by mass of methyl alcohol, 0.6% by mass of cyclohexane, 3.4% by mass of methyl acrylate and 47.5% by mass of water.

On the other hand, the above-mentioned upper layer was effectively used by feeding the upper layer to the distillation column at the middle stage of the distillation column where a 10th stage was provided from the bottom of the distillation column.

A transesterification reaction of methyl acrylate and N,N-dimethylaminoethanol was carried out while properly adding cyclohexane to the flask so that the reaction temperature was 85°C to 102°C. When 4 hours passed from the initiation of the reaction, the transesterification reaction was terminated. After the termination of the reaction, the amount of methyl acrylate contained in the reaction mixture which was obtained by the above-mentioned transesterification reaction was 160 g.

After the termination of the reaction, a reaction mixture obtained by the above-mentioned transesterification reaction was further heated in a reflux ratio of 10 to 15, vapor was taken out from the top of the distillation column, and the vapor was condensed to collect a condensate. At that time, cyclohexane was added to the flask so that the temperature of the reaction mixture included in the flask was maintained to 85°C to 100°C. While taking out the condensate from the top of the distillation column, the temperature at the top of the distillation column gradually increased. The distillation was continued until the temperature at the top of the distillation column attained to a boiling point of cyclohexane of 80°C.

As a result, the content of methyl acrylate in the reaction mixture included in the flask was at most 0.1% by mass. In addition, the condensate was taken out from the top of the distillation column. The amount of the collected condensate was 490 g. This condensate contained 5 g of methanol, 145 g of methyl acrylate and 340 g of cyclohexane. This condensate contained most of the methyl acrylate used in an excessive amount. This collected condensate could be used when a transesterification reaction of methyl acrylate and N,N-dimethylaminoethanol was carried out. From this fact, it was confirmed that loss of methyl acrylate could be prevented by using the above-mentioned condensate when a transesterification reaction of methyl acrylate and N,N-dimethylaminoethanol was carried out.

As a result of the preparation of N,N-dimethylaminoethyl acrylate as described above, the yield of N,N-dimethylaminoethyl acrylate was 852 g (yield on the basis of N,N-dimethylaminoethanol: 96% by mass; yield on the basis of the charged amount of methyl acrylate: 73.8% by mass; yield in the case where methyl acrylate which was collected by the azeotrope with cyclohexane was reused: 93.3% by mass).

From the above results, when the reactor A is used, it can be seen that the condensate can be effectively used by mixing the condensate which was removed from the top of the distillation column with water, separating the resulting mixed solution into two layers, and supplying the upper layer obtained to the distillation column, that the condensate can be effectively used in a new transesterification reaction by further heating the reaction mixture which was obtained by the transesterification reaction, and collecting the condensate from the top of the distillation column, and that an objective N,N-dimethylaminoethyl acrylate can be prepared in a high yield.

Next, as a distillation apparatus B, a 1-liter four-necked flask having a 20-stage Oldershaw distillation column equipped with a condensing apparatus at its top and having a side tube, and an air intake tube was used. The Oldershaw type distillation column was continuously charged with the lower layer obtained in the above at a flow rate of 200 g/h from the middle stage (10th stage) of the distillation column.

A distillation was carried out while refluxing to the top of the distillation column in a reflux ratio of 50, and a part of the condensate obtained in the condensing apparatus was taken out from the condensing apparatus at a flow rate of 22 g/h. As a result, the condensate was composed of 46.3% by mass of methyl alcohol, 32.7% by mass of methyl acrylate and 21.0% by mass of cyclohexane. This condensate was effectively used by returning to the reactor A.

On the other hand, the components of the liquid taken out from the lower stage of the distillation apparatus B were examined. As a result, the liquid was composed of 57.8% by mass of water and 42.2% by mass of methyl alcohol.

Next, as a distillation apparatus C, a 1-liter four-necked flask having a 20-stage Oldershaw distillation column equipped with a condensing apparatus at its top and having a side tube, and an air intake tube was used. The Oldershaw distillation column was continuously charged with the liquid obtained in the above at a flow rate of 184 g/h from the middle stage (10th stage) of the distillation column.

A distillation was carried out while refluxing to the top of the distillation column in a reflux ratio of 1.4, and a part of the condensate obtained in the condensing apparatus was taken out from the condensing apparatus at a flow rate of 76 g/h. As a result, the condensate was composed of 99.5% by mass of methyl alcohol and 0.5% by mass of water. Since this condensate had a high content of methyl alcohol, the condensate could be suitably used as a fuel and in the synthesis of an organic compound.

On the other hand, the components of the liquid taken out from the lower stage of the distillation apparatus C were examined. As a result, the liquid was composed of 100% by mass of water. This water could be suitably used in the separation apparatus of the reactor A.

From the above results, it can be seen that according to Example 12, not only (meth)acrylate which is used as an unreacted raw material and a solvent but also a by-product alcohol and by-product water can be effectively used.

### Comparative Example 1

The same reactor A as used in Example 1 was used. The flask was charged with 694 g (8.06 moles) of methyl acrylate, 552 g (6.20 moles) of N,N-dimethylaminoethanol, 1.76 g of phenothiazine, 22.1 g of dibutyltin oxide and 100 g of isohexane.

Next, a transesterification reaction was carried out at a reaction temperature of 76°C to 93°C while blowing air into the flask at a flow rate of 20 mL/min. More specifically, vapor which was taken out from the top of the distillation column of the reactor was condensed. A part of the resulting condensate was refluxed to the upper part of the distillation column, and the remaining condensate was removed from the reactor. The temperature at the top of the distillation column was controlled to an azeotropic temperature of methyl alcohol and isohexane of 45°C to 56°C by adjusting the amount of the condensate removed from the flask.

The condensate removed from the reactor in an amount of 950 g was mixed with 200 g of water at a temperature of 20°C, and the resulting mixed solution was introduced to a decanter to separate the mixed solution into two layers. The lower layer of the two layers contained methyl alcohol which had been included in the condensate. On the other hand, the upper layer contained isohexane. The upper layer was fed to the 10th stage of the distillation column from the bottom, which was a middle stage of the distillation column.

The transesterification reaction of methyl acrylate and N,N-dimethylaminoethanol was carried out for 13 hours, and the reaction of methyl acrylate and N,N-dimethylaminoethanol was terminated.

At the time of termination of the reaction, the amount of methyl acrylate remaining in the reactor was 160 g.

Next, a distillation was carried out in a reflux ratio of 10 to 15 while adding isohexane to the flask so that the temperature of the reaction mixture included in the flask was 80°C to 90°C, and a condensate was taken out from the top of the distillation column. When the condensate was taken out from the top of the distillation column, the temperature at the top of the distillation column immediately attained to a boiling point of isohexane of 62°C. The amount of the condensate taken out from the top of the distillation column was 300 g.

As a result, the amount of methyl acrylate remaining in the reactor was 10.8% by mass. In addition, 300 g of the condensate taken out from the top of the distillation column contained 5 g of methanol, 5 g of methyl acrylate and 290 g of isohexane. From this fact, it was confirmed that the above-mentioned condensate contained little methyl acrylate which was used as a raw material, and that almost of the methyl acrylate remained in the reactor.

From the above results, the amount of methyl acrylate remaining in the reactor at the time of termination of the reaction was 160 g. In addition, after the termination of the reaction, methyl acrylate did not form an azeotropic mixture together with isohexane, and only 5 g of the methyl acrylate could be collected.

### Comparative Example 2

A 2-liter four-necked flask having a 20-stage Oldershaw distillation column (number of theoretical stages: 15) equipped with a condensing apparatus at its top and having a side tube, and an air intake tube was used as a reactor. The flask of the reactor was charged with 694 g (8.06 moles) of methyl acrylate, 552 g (6.20 moles) of N,N-dimethylaminoethanol, 1.76 g of phenothiazine, 22.1 g of dibutyltin oxide and 100 g of cyclohexane. A transesterification reaction was carried out while blowing air into the flask from the air intake tube at a flow rate of 20 mL/min.

More specifically, vapor which was taken out from the top of the distillation column of the reactor was condensed. A part of the resulting condensate was refluxed to the top of the distillation column, and the remaining condensate was removed from the reactor. The temperature at the top of the distillation column was controlled to an azeotropic temperature of methyl alcohol and cyclohexane of 54°C to 56°C by adjusting the amount of the condensate removed from the flask.

The condensate which was removed from the top of the distillation column of the reactor in an amount of 700 g was mixed with 200 g of water at a temperature of 20°C, and the resulting mixed solution was introduced to a decanter. This mixed solution was separated into two layers of an upper layer and a lower layer. Since methyl alcohol contained in the condensate was extracted with water, the methyl alcohol was contained in the lower layer, and the cyclohexane included in the condensate was contained in the upper layer. The amount of the lower layer was 410 g, and the lower layer contained 47.0% by mass (193 g) of methyl alcohol, 0.5% by mass (2 g) of cyclohexane, 3.6% by mass (15 g) of methyl acrylate and 48.9% by mass (200 g) of water.

A transesterification reaction of methyl acrylate and N,N-dimethylaminoethanol was carried out while properly adding cyclohexane to the flask so that the reaction temperature was 85°C to 102°C. When 4 hours passed from the initiation of the reaction, the transesterification reaction was terminated.

As a result, the yield of N,N-dimethylaminoethyl acrylate was 96.0% by mass, and the yield on the basis of methyl acrylate was 73.8% by mass. In addition, a water layer obtained by extracting methanol from the condensate contained 48.0% by mass of methanol, 0.1% by mass of n-hexane and 3.6% by mass of methyl acrylate, and the amount of methyl acrylate remaining in the reactor was 160 g.

Therefore, according to the method employed in Comparative Example 2, it can be seen that methyl acrylate remains in the reactor in a large amount.

From the above results, according to each of the working examples of the present invention, it can be seen that (meth)acrylate which is used as a raw material remaining after the transesterification reaction of (meth)acrylate which is used as a raw material and an alcohol can be efficiently collected, and that the collected (meth)acrylate which is used as a raw material can be reused when a transesterification reaction of a (meth)acrylate which is used as a raw material and an alcohol is carried out.

### EXPLANATION OF REFERENTIAL NUMBERS

1 reactor A
2 distillation column
3 distillation apparatus B
4 distillation column
5a to 5f pipe
6 condensing apparatus
7 switching apparatus
8 liquid separation apparatus
9 condensing apparatus
10a to 10e pipe
11 switching apparatus
12 collecting unit
13 distillation column
14 distillation apparatus C
15 heating apparatus
16 pipe
17 heating apparatus
18a to 18e pipe
19 condensing apparatus
20 switching apparatus
21 collecting unit

## Claims

1. A system for producing a (meth)acrylate used in producing a (meth)acrylate by transesterification, comprising a reactor A having a distillation column and a distillation apparatus B having a distillation column, wherein
an upper part of the distillation column of the reactor A is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a liquid separation apparatus through a switching apparatus with a pipe for feeding the condensate remaining in the condensing apparatus to the liquid separation apparatus;
an upper part of the liquid separation apparatus is connected with the distillation column through a pipe for refluxing an upper layer of the condensate separated by the liquid separation apparatus to the distillation column; and a lower part of the liquid separation apparatus is connected with the distillation apparatus B through a pipe for feeding a lower layer of the condensate separated by the liquid separation apparatus to the distillation apparatus B;
an upper part of the distillation column of the distillation apparatus B is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a switching apparatus with a pipe for feeding the remaining condensate to the collecting unit; and
a lower part of the distillation apparatus B is connected with a pipe between the switching apparatus and the liquid separation apparatus in the reactor A through a pipe for refluxing a residue existing in the distillation apparatus B to the distillation column of the reactor A.

2. A system for producing a (meth)acrylate used in producing a (meth)acrylate by transesterification, comprising a reactor A having a distillation column, a distillation apparatus B having a distillation column and a distillation apparatus C having a distillation column, wherein
an upper part of the distillation column of the reactor A is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a liquid separation apparatus through the switching apparatus with a pipe for feeding the condensate remaining in the condensing apparatus to the liquid separation apparatus;
an upper part of the liquid separation apparatus is connected with the distillation column through a pipe for refluxing an upper layer of the condensate separated by the liquid separation apparatus to the distillation column; and a lower part of the liquid separation apparatus is connected with the distillation apparatus B through a pipe for feeding a lower layer of the condensate separated by the liquid separation apparatus to the distillation apparatus B;
an upper part of the distillation column of the distillation apparatus B is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a switching apparatus with a pipe for feeding the remaining condensate to the collecting unit;
a lower part of the distillation apparatus B is connected with the distillation apparatus C through a pipe for feeding a residue existing in the distillation apparatus B to the distillation column of the distillation apparatus C;
an upper part of the distillation apparatus C is connected with a condensing apparatus through a pipe; the condensing apparatus is connected with the upper part of the distillation column through a switching apparatus with a pipe for refluxing a part of a condensate obtained in the condensing apparatus to the upper part of the distillation column; and the condensing apparatus is connected with a collecting unit for collecting the condensate remaining in the condensing apparatus through a pipe for feeding the condensate remaining in the condensing apparatus to the collecting unit; and
a lower part of the distillation apparatus C is connected with a pipe between the switching apparatus and the liquid separation apparatus in the reactor A through a pipe for feeding a residue existing in the distillation apparatus C to the distillation column of the reactor A.

## Patentansprüche

1. System zur Herstellung eines (Meth)Acrylats, das bei der Herstellung eines (Meth)Acrylats durch Umesterung verwendet wird, wobei das System einen eine Destillationssäule aufweisenden Reaktor A und eine eine Destillationssäule aufweisende Destillationsvorrichtung B umfasst, wobei
ein oberer Teil der Destillationssäule des Reaktors A mit einer Kondensationsvorrichtung über ein Rohr verbunden ist, die Kondensationsvorrichtung mit dem oberen Teil der Destillationssäule über eine Umschaltvorrichtung mit einem Rohr zum Refluxieren eines Teils eines in der Kondensationsvorrichtung erhaltenen Kondensats zu dem oberen Teil der Destillationssäule verbunden ist, und die Kondensationsvorrichtung mit einer Flüssigkeitstrennvorrichtung über eine Umschaltvorrichtung mit einem Rohr zum Zuführen des in der Kondensationsvorrichtung verbliebenen Kondensats zu der Flüssigkeitstrennvorrichtung verbunden ist;
ein oberer Teil der Flüssigkeitstrennvorrichtung mit der Destillationssäule über ein Rohr zum Refluxieren einer oberen Schicht des durch die Flüssigkeitstrennvorrichtung abgetrennten Kondensats zu der Destillationssäule verbunden ist und ein unterer Teil der Flüssigkeitstrennvorrichtung mit der Destillationsvorrichtung B über ein Rohr zum Zuführen einer unteren Schicht des durch die Flüssigkeitstrennvorrichtung abgetrennten Kondensats zu der Destillationsvorrichtung B verbunden ist;
ein oberer Teil der Destillationssäule der Destillationsvorrichtung B mit einer Kondensationsvorrichtung über ein Rohr verbunden ist, die Kondensationsvorrichtung mit dem oberen Teil der Destillationssäule über eine Umschaltvorrichtung mit einem Rohr zum Refluxieren eines Teils eines in der Kondensationsvorrichtung erhaltenen Kondensats zu dem oberen Teil der Destillationssäule verbunden ist, und die Kondensationsvorrichtung mit einer Sammeleinheit zum Sammeln des in der Kondensationsvorrichtung verbliebenen Kondensats über eine Umschaltvorrichtung mit einem Rohr zum Zuführen des verbliebenen Kondensats zu der Sammeleinheit verbunden ist; und
ein unterer Teil der Destillationsvorrichtung B mit einem Rohr zwischen der Umschaltvorrichtung und der Flüssigkeitstrennvorrichtung in dem Reaktor A über ein Rohr zum Refluxieren eines in der Destillationsvorrichtung B vorhandenen Rests zu der Destillationssäule des Reaktors A verbunden ist.

2. System zur Herstellung eines (Meth)Acrylats, das bei der Herstellung eines (Meth)Acrylats durch Umesterung verwendet wird, wobei das System einen eine Destillationssäule aufweisenden Reaktor A, eine eine Destillationssäule aufweisende Destillationsvorrichtung B und eine Destillationsvorrichtung C, die eine Destillationssäule aufweist, umfasst, wobei
ein oberer Teil der Destillationssäule des Reaktors A mit einer Kondensationsvorrichtung über ein Rohr verbunden ist, die Kondensationsvorrichtung mit dem oberen Teil der Destillationssäule über eine Umschaltvorrichtung mit einem Rohr zum Refluxieren eines Teils eines in der Kondensationsvorrichtung erhaltenen Kondensats zu dem oberen Teil der Destillationssäule verbunden ist, und die Kondensationsvorrichtung mit einer Flüssigkeitstrennvorrichtung über eine Umschaltvorrichtung mit einem Rohr zum Zuführen des in der Kondensationsvorrichtung verbliebenen Kondensats zu der Flüssigkeitstrennvorrichtung verbunden ist;
ein oberer Teil der Flüssigkeitstrennvorrichtung mit der Destillationssäule über ein Rohr zum Refluxieren einer oberen Schicht des durch die Flüssigkeitstrennvorrichtung abgetrennten Kondensats zu der Destillationssäule verbunden ist und ein unterer Teil der Flüssigkeitstrennvorrichtung mit der Destillationsvorrichtung B über ein Rohr zum Zuführen einer unteren Schicht des durch die Flüssigkeitstrennvorrichtung abgetrennten Kondensats zu der Destillationsvorrichtung B verbunden ist;
ein oberer Teil der Destillationssäule der Destillationsvorrichtung B mit einer Kondensationsvorrichtung über ein Rohr verbunden ist, die Kondensationsvorrichtung mit dem oberen Teil der Destillationssäule über eine Umschaltvorrichtung mit einem Rohr zum Refluxieren eines Teils eines in der Kondensationsvorrichtung erhaltenen Kondensats zu dem oberen Teil der Destillationssäule verbunden ist, und die Kondensationsvorrichtung mit einer Sammeleinheit zum Sammeln des in der Kondensationsvorrichtung verbliebenen Kondensats über eine Umschaltvorrichtung mit einem Rohr zum Zuführen des verbliebenen Kondensats zu der Sammeleinheit verbunden ist;
ein unterer Teil der Destillationsvorrichtung B mit der Destillationsvorrichtung C über ein Rohr zum Zuführen eines in der Destillationsvorrichtung B vorhandenen Restes zu der Destillationssäule der Destillationsvorrichtung C verbunden ist;
ein oberer Teil der Destillationsvorrichtung C mit einer Kondensationsvorrichtung über ein Rohr verbunden ist, die Kondensationsvorrichtung mit dem oberen Teil der Destillationssäule über eine Umschaltvorrichtung mit einem Rohr zum Refluxieren eines Teils eines in der Kondensationsvorrichtung erhaltenen Kondensats zu dem oberen der Destillationssäule verbunden ist, und die Kondensationsvorrichtung mit einer Sammeleinheit zum Sammeln des in der Kondensationsvorrichtung verbliebenen Kondensats über ein Rohr zum Zuführen des in der Kondensationsvorrichtung verbliebenen Kondensats zu der Sammeleinheit verbunden ist; und
ein unterer Teil der Destillationsvorrichtung C mit einem Rohr zwischen der Umschaltvorrichtung und der Flüssigkeitstrennvorrichtung in dem Reaktor A über ein Rohr zum Zuführen eines in der Destillationsvorrichtung C vorhandenen Rests zu der Destillationssäule des Reaktors A verbunden ist.

## Revendications

1. Système permettant de produire un méthacrylate utilisé dans la production d'un méthacrylate par transestérification, comprenant un réacteur A possédant une colonne de distillation et un appareil de distillation B possédant une colonne de distillation, dans lequel
une partie supérieure de la colonne de distillation du réacteur A est reliée à un appareil de condensation grâce à un conduit, l'appareil de condensation est relié à la partie supérieure de la colonne de distillation grâce à un appareil de commutation équipé d'un conduit pour le reflux d'une partie d'un condensat obtenu dans l'appareil de condensation à la partie supérieure de la colonne de distillation ; et l'appareil de condensation est relié à un appareil de séparation de liquide grâce à un appareil de commutation équipé d'un conduit pour alimenter le condensat restant dans l'appareil de condensation à l'appareil de séparation de liquide ;
une partie supérieure de l'appareil de séparation de liquide est reliée à la colonne de distillation grâce à un conduit pour le reflux d'une partie supérieure du condensat séparée au moyen de l'appareil de séparation du liquide à la colonne de distillation ; et une partie inférieure de l'appareil de séparation de liquide est reliée à l'appareil de distillation B grâce à un conduit pour alimenter une partie inférieure du condensat séparée au moyen de l'appareil de séparation de liquide à l'appareil de distillation B ;
une partie supérieure de la colonne de distillation de l'appareil de distillation B est reliée à un appareil de condensation grâce à un conduit ; l'appareil de condensation est relié à la partie supérieure de la colonne de distillation grâce à un appareil de commutation équipé d'un conduit pour le reflux d'une partie d'un condensat obtenu dans l'appareil de condensation à la partie supérieure de la colonne de distillation ; et l'appareil de condensation est relié à une unité de collection pour la collecte du condensat restant dans l'appareil de condensation grâce à un appareil de commutation équipé d'un conduit pour alimenter le condensat restant à l'unité de collecte ; et
une partie inférieure de l'appareil de distillation B est reliée au moyen d'un conduit entre l'appareil de commutation et l'appareil de séparation du liquide dans le réacteur A grâce à un conduit pour le reflux d'un résidu existant dans l'appareil de distillation B à la colonne de distillation du réacteur A.

2. Système permettant de produire un méthacrylate utilisé dans la production d'un méthacrylate par transestérification, comprenant un réacteur A possédant une colonne de distillation et un appareil de distillation B possédant une colonne de distillation et un appareil de distillation C possédant une colonne de distillation, dans lequel une partie supérieure de la colonne de distillation du réacteur A est reliée à un appareil de condensation grâce à un conduit ; l'appareil de condensation est relié à la partie supérieure de la colonne de distillation à travers un appareil de commutation équipé d'un conduit pour le reflux d'une partie d'un condensat obtenu dans l'appareil de condensation à la partie supérieure de la colonne de distillation ; et l'appareil de condensation est relié à un appareil de séparation de liquide à travers l'appareil de commutation équipé d'un conduit pour alimenter le condensat restant dans l'appareil de condensation à l'appareil de séparation de liquide ;
une partie supérieure de l'appareil de séparation du liquide est reliée à la colonne de distillation grâce à un conduit pour le reflux d'une couche supérieure condensat séparée au moyen de l'appareil de séparation du liquide à la colonne de distillation ; et une partie inférieure de l'appareil de séparation du liquide est reliée à l'appareil de distillation B grâce à un conduit pour alimenter une couche inférieure du condensat séparée au moyen de l'appareil de séparation du liquide à l'appareil de distillation B ;
une partie supérieure de la colonne de distillation de l'appareil de distillation B est reliée à un appareil de condensation grâce à un conduit ; l'appareil de condensation est relié à la partie supérieure de la colonne de distillation grâce à un appareil de commutation équipé d'un conduit pour le reflux d'une partie d'un condensat obtenu dans l'appareil de condensation à la partie supérieure de la colonne de distillation ; et l'appareil de condensation est relié à une unité de collection pour la collecte du condensat restant dans l'appareil de condensation grâce à un appareil de commutation équipé d'un conduit pour alimenter le condensat restant à l'unité de connexion ;
une partie inférieure de l'appareil de distillation B est reliée à l'appareil de distillation C grâce à un conduit pour alimenter un résidu existant dans l'appareil de distillation B à la colonne de distillation de l'appareil de distillation C ;
une partie supérieure de l'appareil de distillation C est reliée à un appareil de condensation grâce à un conduit ; l'appareil de condensation est relié à la partie supérieure de la colonne de distillation grâce à un appareil de commutation équipé d'un conduit pour le reflux d'une partie d'un condensat obtenu dans l'appareil de condensation à la partie supérieure de la colonne de distillation ; et l'appareil de condensation est relié à une unité de collection pour la collecte du condensat restant dans l'appareil de condensation grâce à un conduit pour alimenter le condensat restant dans l'appareil de condensation à l'unité de collection ; et
une partie inférieure de l'appareil de distillation C est reliée à un conduit entre l'appareil de commutation et l'appareil de séparation de liquide dans le réacteur A grâce à un conduit pour alimenter un résidu existant dans l'appareil de distillation C à la colonne de distillation du réacteur A.
